# EUROPÄISCHE PATENTSCHRIFT

(11) **EP 0 515 940 B1**
(45) Veröffentlichungstag und Bekanntmachung des Hinweises auf die Patenterteilung: **17.07.1996**
(21) Anmeldenummer: 92108324.2
(22) Anmeldetag: 18.05.1992
(51) Int. Cl.: C07D 401/04, C07D 211/90, C07D 215/12, C07D 215/14, A61K 31/445, C07D 261/06, C07D 261/08

(54) **Neue 2-Amino-5-cyano-1,4-dihydropyridine, Verfahren zu ihrer Herstellung und ihre Verwendung in Arzneimitteln**
New 2-amino-5-cyano-1,4-dihydropyridines, process for their preparation and use in pharmaceutical compositions
2-Amino-5-cyano-1,4-dihydropyridines, procédé pour leur préparation et leur utilisation en tant que médicaments

(30) Priorität: 30.05.1991 DE 4117750
(43) Veröffentlichungstag der Anmeldung: 02.12.1992
(73) Patentinhaber: BAYER AG, 51368 Leverkusen (DE)
(72) Erfinder: Stoltefuss, Jürgen, Dipl.-Ing., W-5657 Haan 2 (DE); Goldmann, Siegfried, Dr., W-5600 Wuppertal 11 (DE); Straub, Alexander, Dr., W-5600 Wuppertal 1 (DE); Böshagen, Horst, Dr., W-5657 Haan (DE); Bechem, Martin, Dr., W-5600 Wuppertal 1 (DE); Gross, Rainer, Prof. Dr., W-5600 Wuppertal 1 (DE); Hebisch, Siegbert, Dr., W-5600 Wuppertal 1 (DE); Hütter, Joachim, Dr., W-5600 Wuppertal 12 (DE); Rounding, Howard-Paul, Dr., W-5600 Wuppertal 1 (DE)

(56) Entgegenhaltungen:
- EP-A- 0 026 317
- EP-A- 0 071 819
- EP-A- 0 452 712
- DE-A- 2 623 170
- DE-A- 2 658 804
- J. HETEROCYCLIC CHEMISTRY Bd. 6, Nr. 2, April 1969, Seiten 243 - 245; J. B. WOMMCK ET. AL.: 'THE SYNTHESIS OF QUINOLINE AND ISOQUINOLINECARBOXALDEHYDES'
- JOURNAL OF THE CHEMICAL SOCIETY. 1946, LETCHWORTH GB Seiten 884 - 888; V. A. PETROW: 'NEW SYNTHESES OF HETEROCYCLIC COMPOUNDS'

## Beschreibung

Die vorliegende Erfindung betrifft neue 2-Amino-5-cyano-4-chinolin-1,4-dihydropyridine, Verfahren zu ihrer Herstellung und ihre Verwendung in Arzneimitteln, insbesondere in Mitteln zur Behandlung von Herz-Kreislauferkrankungen.

Es ist bereits bekannt geworden, daß einige 2- und 6-Amino-3,4-dihydropyridine neben einer antiarrhythmischen auch eine lipidabsorptionshemmende Wirkung besitzen (vgl. EP 73 997).

Ferner sind 2-Amino-1,4-dihydropyridine teilweise auch mit einer vasodilatorischen und antihypertensiven Wirkung beschrieben (vgl. z. B. DE 2 242 786 und DE 2 210 674). In EP-A-0 026 317 und DE-A-2 658 804 werden bereits 3- bzw. 5-Cyano-dihydropyridine beschrieben, die sich chemisch von den erfindungsgemäßen Verbindungen dadurch unterscheiden, daß sie keine Aminosubstitution in 2-Position und keinen Chinolinrest in 4-Position tragen und in pharmakologischer Hinsicht dadurch verschieden sind, daß sie eine calciumantagonistische Wirkung bzw. negativ inotrope Wirkung besitzen. In der DOS 2 117 571 werden Dihydropyridine mit calciumantagonistischer Wirkung beschrieben, in deren allgemeiner Substituentendefinition in 4-Position auch Chinolin genannt wird. Im Unterschied zu den erfindungsgemäßen Verbindungen fehlen hier 2-Amino- und die 5-Cyano-Substituenten.

Bei Kenntnis dieser Eigenschaften der Dihydropyridine war es nicht vorhersehbar, daß die erfindungsgemäßen Verbindungen eine kontraktionskraftverstärkende, am Herzmuskel positiv inotrope Wirkung mit weitgehend gefäßneutralem Verhalten besitzen.

Einige der erfindungsgemäßen Verbindungen der Formel (I) fallen unter den allgemeinen Anspruch in EP 71 819, ohne daß dort jedoch ein konkreter Stoffvertreter genannt ist. Dieses Dokument enthält keinen Hinweis darauf, daß 4-Chinolin-dihydropyridine, die gleichzeitig eine Aminogruppe in 2-Position und eine Cyanogruppe in 5-Position tragen, besonders wertvolle calciumagonistische Wirkstoffe sind.

Die vorliegende Erfindung betrifft 2-Amino-5-cyano-4-chinolin-1,4-dihydropyridine der allgemeinen Formel (I) 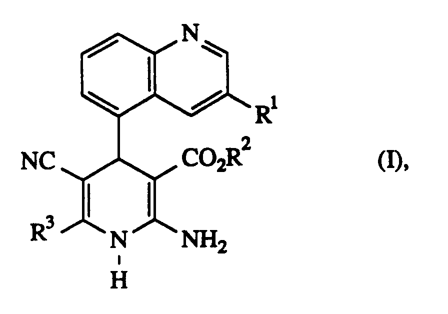
in welcher
- R¹: für Aryl mit 6 bis 10 Kohlenstoffatomen steht, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel -NR⁴R⁵ substituiert ist,
worin
- R⁴ und R⁵: gleich oder verschieden sind und Wasserstoff, geradkettiges oder verzweigtes Alkyl mit bis zu 8 Kohlenstoffatomen, Phenyl oder Benzyl bedeuten,
oder für Thienyl steht,
- R²: für Wasserstoff steht, oder
für Cycloalkyl mit 5 bis 8 Kohlenstoffatomen steht oder
für geradkettiges oder verzweigtes Alkyl, Alkenyl, Alkadienyl oder Alkinyl mit jeweils bis zu 10 Kohlenstoffatomen steht, die gegebenenfalls 1- oder 2-fach gleich oder verschieden durch Halogen, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 8 Kohlenstoffatomen oder durch Cycloalkyl mit 3 bis 8 Kohlenstoffatomen, Phenoxy oder Phenyl substituiert sind, wobei die letzteren ihrerseits bis zu 2-fach gleich oder verschieden durch Halogen, durch geradkettiges oder verzweigtes Alkyl oder Alkoxy mit jeweils bis zu 6 Kohlenstoffatomen substituiert sein können,
oder durch die Gruppe -NR⁴R⁵ substituiert sind,
worin
R⁴ und R⁵ die oben angegebene Bedeutung haben,
- R³: für Wasserstoff oder geradkettiges oder verzweigtes Alkyl mit bis zu 8 Kohlenstoffatomen steht,
und deren physiologisch unbedenklichen Salze.

Physiologisch unbedenkliche Salze sind Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),
in welcher
- R¹: für Phenyl steht, das gegebenenfalls bis zu 3-fach gleich oder verschieden durch Halogen, Nitro, Cyano, Trifluormethyl oder durch geradkettiges oder verzweigtes Alkyl oder Alkoxy mit jeweils bis zu 6 Kohlenstoffatomen, Benzyl oder durch eine Gruppe der Formel -NR⁴R⁵ substituiert ist,
worin
- R⁴ und R⁵: gleich oder verschieden sind und Wasserstoff, geradkettiges oder verzweigtes Alkyl mit bis zu 6 Kohlenstoffatomen, Phenyl oder Benzyl bedeuten,
oder für Thienyl steht,
- R²: für Wasserstoff steht,
für Cyclopentyl oder Cyclohexyl steht oder
für geradkettiges oder verzweigtes Alkyl oder Alkenyl mit jeweils bis zu 10 Kohlenstoffatomen steht, die gegebenenfalls durch Halogen, Hydroxy, Carboxy, Cyano oder durch geradkettiges oder verzweigtes Alkylthio, Alkoxycarbonyl, Alkoxy, Acyl oder Acyloxy mit jeweils bis zu 6 Kohlenstoffatomen, Cyclopropyl, Cyclopentyl, Cyclohexyl, Phenoxy oder Phenyl substituiert sind, wobei die beiden letzteren durch Halogen, Methyl, Methoxy oder Ethoxy substituiert sein können oder durch die Gruppe -NR⁴R⁵ substituiert sind,
worin
R⁴ und R⁵ die oben angegebene Bedeutung haben,
- R³: für Wasserstoff oder geradkettiges oder verzweigtes Alkyl mit bis zu 6 Kohlenstoffatomen steht
und deren physiologisch unbedenklichen Salze.

Besonders bevorzugt sind Verbindungen der allgemeinen Formel (I),
in welcher
- R¹: für Phenyl steht, das gegebenenfalls bis zu 2-fach gleich oder verschieden durch Fluor, Chlor, Nitro, Trifluormethyl oder durch geradkettiges oder verzweigtes Alkyl oder Alkoxy mit jeweils bis zu 4 Kohlenstoffatomen oder durch eine Gruppe der Formel -NR⁴R⁵ substituiert ist,
worin
- R⁴ und R⁵: gleich oder verschieden sind und Wasserstoff oder geradkettiges oder verzweigtes Alkyl mit bis zu 4 Kohlenstoffatomen, Phenyl oder Benzyl bedeuten,
oder für Thienyl steht,
- R²: für Wasserstoff steht, oder
für Cyclopentyl steht,
für geradkettiges oder verzweigtes Alkyl mit bis zu 8 Kohlenstoffatomen steht, das gegebenenfalls durch Fluor, Chlor, Hydroxy, Carboxy, Cyano oder durch geradkettiges oder verzweigtes Alkoxycarbonyl, Alkoxy oder Acyloxy mit jeweils bis zu 4 Kohlenstoffatomen, Phenyl, Phenoxy, Cyclopropyl, Cyclopentyl, Cyclohexyl oder durch die Gruppe -NR⁴R⁵ substituiert ist,
worin
R⁴ und R⁵ die oben angegebene Bedeutung haben,
- R³: für Wasserstoff oder für geradkettiges oder verzweigtes Alkyl mit bis zu 4 Kohlenstoffatomen steht,
und deren physiologisch unbedenklichen Salze.

Die Herstellung der erfindungsgemäßen Verbindungen der allgemeinen Formel (I) ist dadurch gekennzeichnet, daß man
[A] entweder Aldehyde der allgemeinen Formel (II) 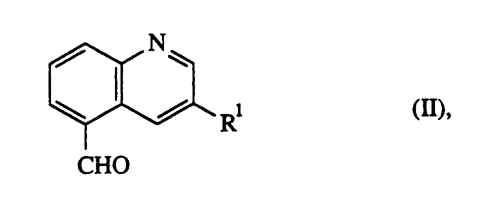 in welcher
   R¹ die oben angegebene Bedeutung hat,
   direkt mit Verbindungen der allgemeinen Formel (III) 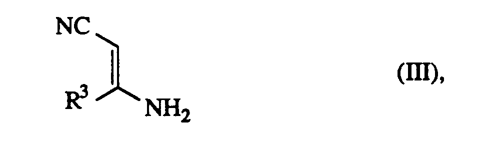 in welcher
   R³ die oben angegebene Bedeutung hat,
   und Verbindungen der tautomeren Formeln (IV) oder (IVa) 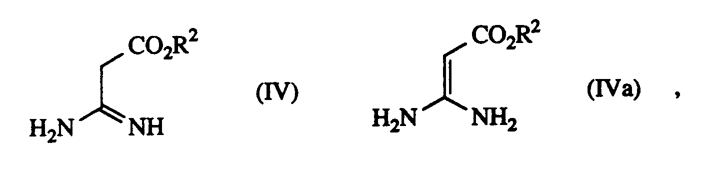 in welcher
   R² die oben angegebene Bedeutung hat,
   in inerten Lösemittteln bei Temperaturen zwischen 10°C und 150°C umsetzt,
   oder
[B] Ylidenverbindungen der allgemeinen Formel (V) 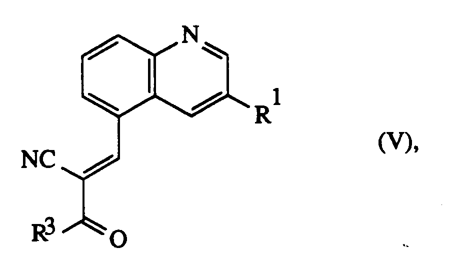 in welcher
   R¹ und R³ die oben angegebene Bedeutung haben,
   mit Verbindungen der allgemeinen Formel (VI) bzw. (VIa) 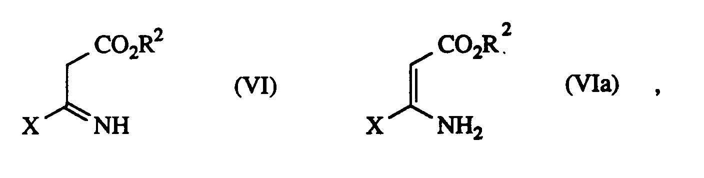 in welcher
   R² die oben angegebene Bedeutung hat, und
   - X: für die Aminogruppe oder die Gruppe OR⁶ steht, wobei
   - R⁶: für C₁-C₄-Alkyl steht
   gegebenenfalls in Anwesenheit von inerten organischen Lösemitteln bei Temperaturen von 10°C bis 150°C umsetzt, wobei für den Fall, daß X für die Gruppe OR⁶ steht, Ammoniumsalze, wie Ammoniumacetat zugegeben werden.

Im Fall der reinen Enantiomeren wird entweder das entstehende Diastereomerengemisch der jeweiligen Verbindungen der allgemeinen Formel (I), in welcher R² für einen definierten chiralen Rest steht, zunächst getrennt, dann in die entsprechenden Carbonsäuren (R² = H) überführt und in einem letzten Schritt verestert oder die jeweiligen Diastereomere werden direkt mit den entsprechenden Alkoholen, insbesondere in Form der Alkoholate umgeestert.

Die erfindungsgemäßen Verfahren können durch folgendes Formelschema beispielhaft erläutert werden: 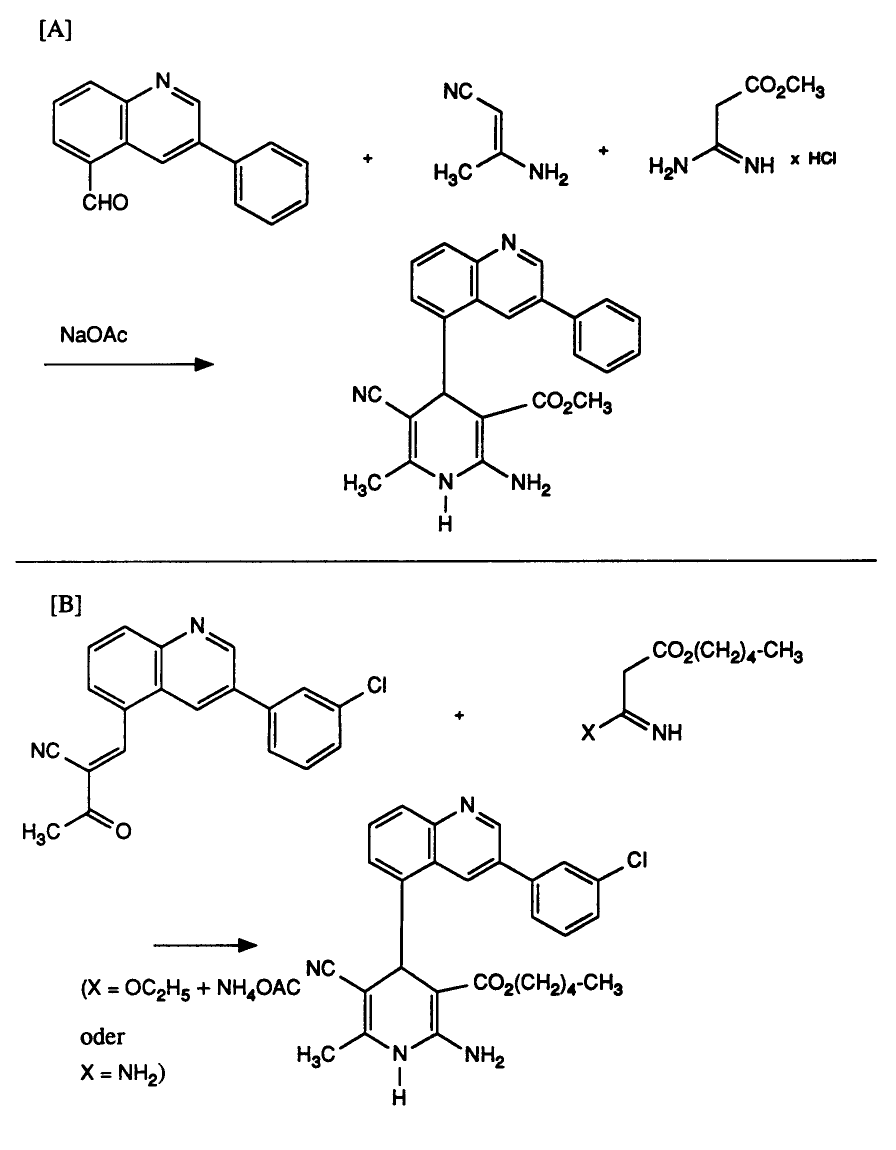
Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Diethylenglykoldimethylether, Acetonitril, oder Amide wie Hexamethylphosphorsäuretriamid oder Dimethylformamid, oder Essigsäure, Alkylacetate oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind in Abhängigkeit von der jeweiligen Verfahrensvariante [A] oder [B] Methanol, Isopropanol, Ethanol und n-Propanol, Acetonitril oder Tetrahydrofuran.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzung kann bei Normaldruck, aber auch bei erhöhtem oder erniedrigtem Druck (z.B. 0,5 bis 3 bar) durchgeführt werden. Im allgemeinen arbeitet man bei Normaldruck.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate, Hydroxyaminosäurederivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Verbindungen der allgemeinen Formel (II) sind teilweise bekannt und können nach üblichen Methoden hergestellt werden, indem man z.B. die entsprechenden Alkyl- oder Hydroxyalkyl-chinoline oxidiert oder die entsprechenden Carboxychinoline reduziert (vgl. auch DOS 4 011 105).

Alternativ kann auch 4-Amino-3-hydroxyphthalid, das durch übliche Hydrierung des literaturbekannten 4-Nitro-3-hydroxyphthalid [vgl. T. Watanabe et al., Chem. Pharm. Bull. 20 (10), 2123 - 2127 (1970)] in Anwesenheit eines Katalysators, bevorzugt mit Palladium/Bariumsulfat, erhalten wird, mit Verbindungen der allgemeinen Formel R¹-CH₂-CHO, die teilweise bekannt sind [vgl. z.B. Beilstein 7, 292], zu Verbindungen der allgemeinen Formel (II) über die entsprechenden Carbonsäuren umgesetzt werden.

Die Verbindungen der allgemeinen Formel (III), (IV) und (IVa) sind an sich bekannt oder können nach literaturbekannten Methoden hergestellt werden [vgl. J. Heterocycl. Chem. 26, 1575 (1989); Liebigs Ann. Chem. 1977, 1895 - 1908].

Die Ylidenverbindungen der allgemeinen Formel (V) sind neu und können hergestellt werden, indem man Verbindungen der allgemeinen Formel (VII) 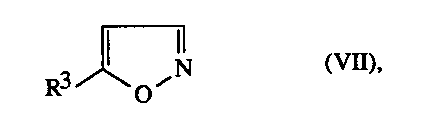
in welcher
R³ die oben angegebene Bedeutung hat,
mit Alkalihydroxiden oder Alkalialkoholaten in die Alkalisalze der Verbindungen der allgemeinen Formel (VIII)

R³-CO-CH₂-CN (VIII),

in welcher
R³ die oben angegebene Bedeutung hat,
überführt,
und diese entweder in situ oder nach Isolation, mit Aldehyden der allgemeinen Formel (II) in einem der oben aufgeführten inerten Lösemitteln, vorzugsweise in Alkoholen, Essigester, Methylenchlorid, Acetonitril, Chloroform oder Ethern, unter Zusatz von Säure, vorzugsweise Essigsäure und gegebenenfalls in Anwesenheit eines Katalysators, beispielsweise Piperidin-acetat bei Temperaturen zwischen 0°C und 150°C, vorzugsweise zwischen 20°C und 110°C umsetzt.

Die Verbindungen der allgemeinen Formel (VII) sind ebenfalls größtenteils bekannt oder können nach üblicher Methode hergestellt werden [vgl. Helv. Chim. Acta, Vol. XLVU, Fasciculens II (1963), Nr. 56 - 57, S. 543 - 551].

Die Verbindungen der allgemeinen Formel (VIII) sind ebenfalls bekannt oder können nach literaturbekannten Methoden hergestellt werden.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Vorzugsweise zeigen sie eine positiv ionotrope Wirkung. Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. als Perfusionsmedium dient eine Krebs-Henseleit-Lösung (1) (118,5 mmol/l NaCl, 4,75 mmol/l KCl, 1,19 mmol/l KH₂PO₄, 1,19 mmol/l MgSO₄, 25 mmol/l NaHCO₃, 0,013 mmol/l Na₂EDTA), deren CaCl₂-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% O₂, 5% CO₂) zur Aufrechterhaltung des pH-Wertes 7,4 begast. Die Herzen werden mit konstantem Fluß (10 ml/min) bei 32°C mittels einer Rollenquetschpumpe perfundiert.

Zur Messung der Herzfünktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert (Opie, L., J. Physiol. 180 (1965), 529 -541). Der Perfusionsdruck wird mittels eines Druckaufnehmers, der vor dem Herzen mit dem Perfusionssystem in Verbindung steht, registriert. Unter diesen Bedingungen zeigt eine Senkung des Perfüsionsdrucks eine Koronardilatation, eine Zu-bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Substanzeffekte auf die Kontraktionsamplitude isolierter Meerschweinchen-Herzvorhöfe bei einer Wirkstoffkonzentration von 10:4 g/l.

| Bsp.-Nr. | Kontraktionskraft (% Kontrolle) |
|---|---|
| 6 | +14 |
| 7 | +103 |
| 13 | +21 |
| 14 | +43 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindung

### Beispiel I

### 1-(3-Phenylchinol-5-yliden)-3-oxo-buttersäurenitril

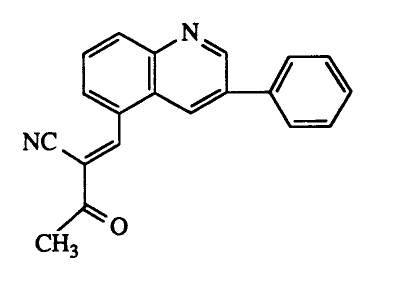

15,45 g (66,3 mMol) 3-Phenyl-chinolin-5-aldehyd werden in 420 ml Dichlormethan mit 6,97 g (66,4 mMol) 3-Oxo-buttersäurenitril-Natriumsalz (erhalten durch Lösen von 5-Methylisoxazol in der äquivalenten Menge Natriummethylat-Lösung und Einengen der Lösung), 4,34 ml Essigsäure und 0,66 ml Piperidin 24 Stunden am Wasserabscheider gekocht. Es wird abgekühlt, 2 x mit Wasser gewaschen, von etwas ausgefallenem Nebenprodukt filtriert und eingeengt. Das gewünschte Produkt kristallisiert beim Verrühren mit Ethanol. Man erhält 12,7 g gelbe Kristalle vom Schmelzpunkt 142°C bis 144°C.

### Herstellungsbeispiele

### Beispiel 1 (Verfahren B)

### 2-Amino-1,4-dihydropyridin-5-cyano-6-methyl-4-(3-phenylchinolin-5-yl)-3-carbonsäure-(S)-(1-methoxycarbonyl)-ethylester

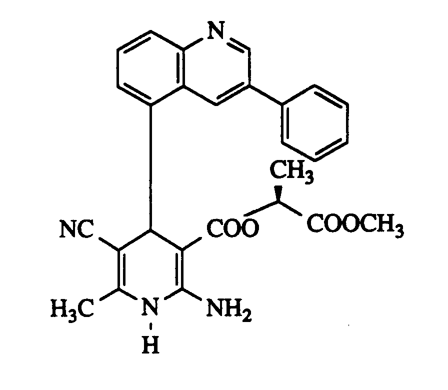

### Methode A

2,5 g (8,4 mmol) 1-(3-Phenylchinol-5-yliden)-3-oxo-buttersäurenitril werden in 15 ml Isopropanol mit 2,2 g (10,1 mmol) 3-Imino-3-ethoxy-essigsäure-(S)-(1-methoxycarbonyl)-ethylester und 1,43 g (18,44 mmol) Ammoniumacetat über Nacht gekocht. Es wird eingeengt, in Essigester aufgenommen, 2 mal mit Wasser gewaschen, getrocknet und eingeengt Das erhaltene Diasteromerengemisch wird über eine Kieselgelsäule mit Methylenchlorid/Essigester-Gemischen von 10:1 bis 2:1 getrennt, die reinen Fraktionen werden gesammelt und eingeengt. Man erhält 1,25 g des Diastereomeren A (R_{f}-Wert = 0,27; DC-Alurolle, Merck, Kieselgel 60, F 254) und 778 mg des kristallinen Diastereomeren B vom Schmp.: 274-276°C und R_{f}-Wert 0,20.

### Methode B

2,5 g (8,4 mmol) 1-(3-Phenylchinol-5-yliden)-3-oxo-buttersäurenitril werden in 10 ml Methylacetat mit 2,2 g (10,1 mmol) (S)-1-Methoxycarbonyl-ethyl-3-imino-3-ethoxyacetat und 1,43 g (18,44 mmol) Ammoniumacetat für 3 Stunden unter Rückfluß gekocht. Nach Abkühlen werden die ausgefallenen Kristalle abgefiltert. Man erhält 1,1 g des farblosen Diastereomeren B vom Schmelzpunkt 272-274°C.

### Beispiel 2

### (-)-2-Amino-1,4-dihydropyridin-5-cyano-6-methyl-4-(3-phenylchinolin-5-yl)-3-carbonsäureethylester

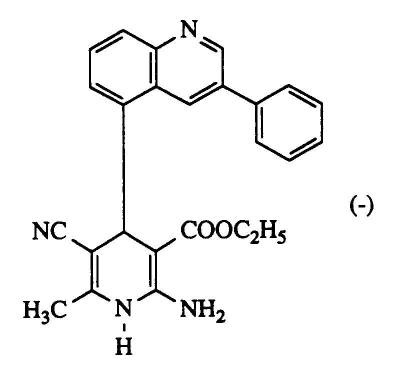

In eine Lösung von 200 mg (28,3 mmol) Lithium in 25 ml Ethanol werden 1,3 g (2,76 mmol) des Diastereomeren B aus Beispiel 1 gegeben. Es wird 30 Minuten zum Sieden erhitzt, abgekühlt und eingeengt. Der Eindampfrückstand wird in Essigester/Wasser aufgenommen, getrennt, die organische Phase wird zweimal mit Wasser gewaschen und eingeengt. Das erhaltene Produkt wird durch Flash-Chromatographie gereinigt und mit Acetonitril kristallisiert. Man erhält 678 mg farblose Kristalle vom Schmelzpunkt 195-198°C.

### Beispiel 3 (Verfahren A)

### 2-Amino-1,4-dihydro-5-cyano-6-methyl-4-(3-phenylchinolin-5-yl)pyridin-3-carbonsäureisopropylester

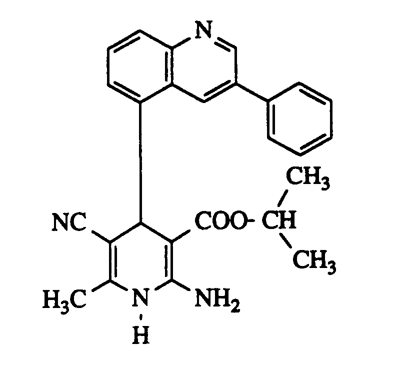

4,66 g (20 mmol) 3-Phenyl-chinolin-5-aldehyd werden in 40 ml Isopropanol mit 1,64 g (20 mmol) 3-Aminocrotonsäurenitiil, 3,6 g (20 mmol) Amidinoessigsäureisopropylester-hydrochlorid und 1,64 g (20 mmol) Natriumacetat 20 Stunden gekocht. Es wird eingeengt, in Essigester/Wasser aufgenommen und abgetrennt. Die organische Phase wird 2 mal mit Wasser gewaschen, getrocknet und eingeengt. Das Reaktionsgemisch wird über eine Kieselgelsäule mit Methylenchlorid/Essigester-Gemischen getrennt. Die gewünschten Fraktionen werden gesammelt und gereinigt. Der erhaltene Eindampfrückstand wird mit Acetonitril kristallisiert und abgesaugt. Man erhält 635 mg farblose Kristalle vom Schmelzpunkt 227-229°C.

### Beispiel 4

### 2-Amino-1,4-dihydropyridin-5-cyano-6-methyl-4-(3-(3-chlorphenyl)-chinolin-5-yl)-carbonsäure-isopropylester

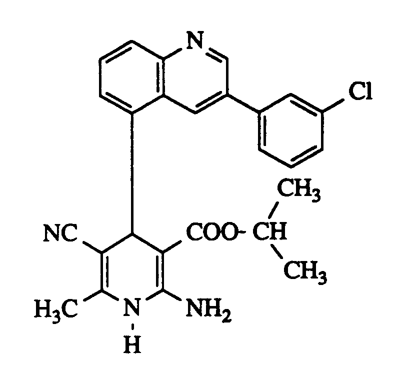

1,66 g (5 mmol) 1-[3-(3-Chlorphenyl)-chinol-5-yliden]-3-oxo-buttersäure-n itril werden in 10 ml Isopropanol mit 905 mg (5 mmol) Amidinoessigsäureisopropylester-hydrochlorid und 410 mg (5 mmol) Natriumacetat über Nacht zum Rückfluß erhitzt. Es wird abgekühlt und eingeengt. Der erhaltene Eindampfrückstand wird in Essigester/Wasser gelöst, getrennt, die organischen Phase wird mit Natriumhydrogencarbonat-Lösung und 2 mal mit Wasser gewaschen, getrocknet und eingeengt. Durch Verrühren mit Acetonitril erhält man farblose Kristalle vom Schmp. 264-265°C.

### Beispiel 5 (Verfahren B ohne Isolierung des Zwischenproduktes)

### 2-Amino-1,4-dihydro-5-cyano-6-methyl-4-(3-phenylchinolin-5-yl)-pyridin-3-carbonsäure-n-propylester

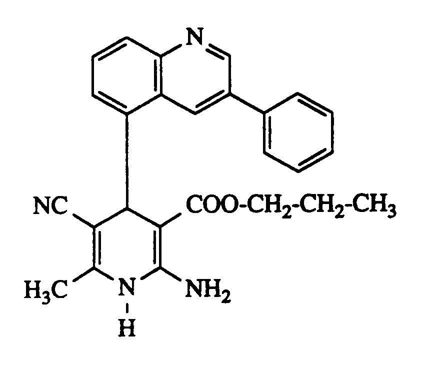

23,3 g (100 mmol) 3-Phenyl-chinolin-5-aldehyd werden in 200 ml n-Propanol suspendiert und mit 8,25 ml (100 mmol) 5-Methylisoxazol verrührt. Es wird eine Lösung von 2,3 g Natrium in 100 ml n-Propanol zugetropft und 4 Stunden bei 40-50°C gerührt. Dabei wird eine braune Lösung erhalten. Es werden 18,04 g (100 mmol) Amidinoessigsäurepropylester-hydrochlorid und 6 ml Essigsäure (100 mmol) zugegeben und 20 Stunden gekocht. Es wird eingeengt, in Essigester/Wasser gelöst und getrennt. Die Essigesterphase wird mit Natriumhydrogencarbonat-Lösung und 2 mal mit Wasser gewaschen, getrocknet und eingeengt. Der erhaltene Rückstand wird in 100 ml heißem Acetonitril gelöst und kristallisieren gelassen. Es wird abgesaugt und aus n-Propanol, dann aus Acetonitril umkristallisiert. Man erhält 6,9 g farblose Kristalle vom Schmelzpunkt 237°C.

Die in der Tabelle 1 aufgeführten Beispiele werden in Analogie zu den Vorschriften der Beispiele 1 bis 5 hergestellt:

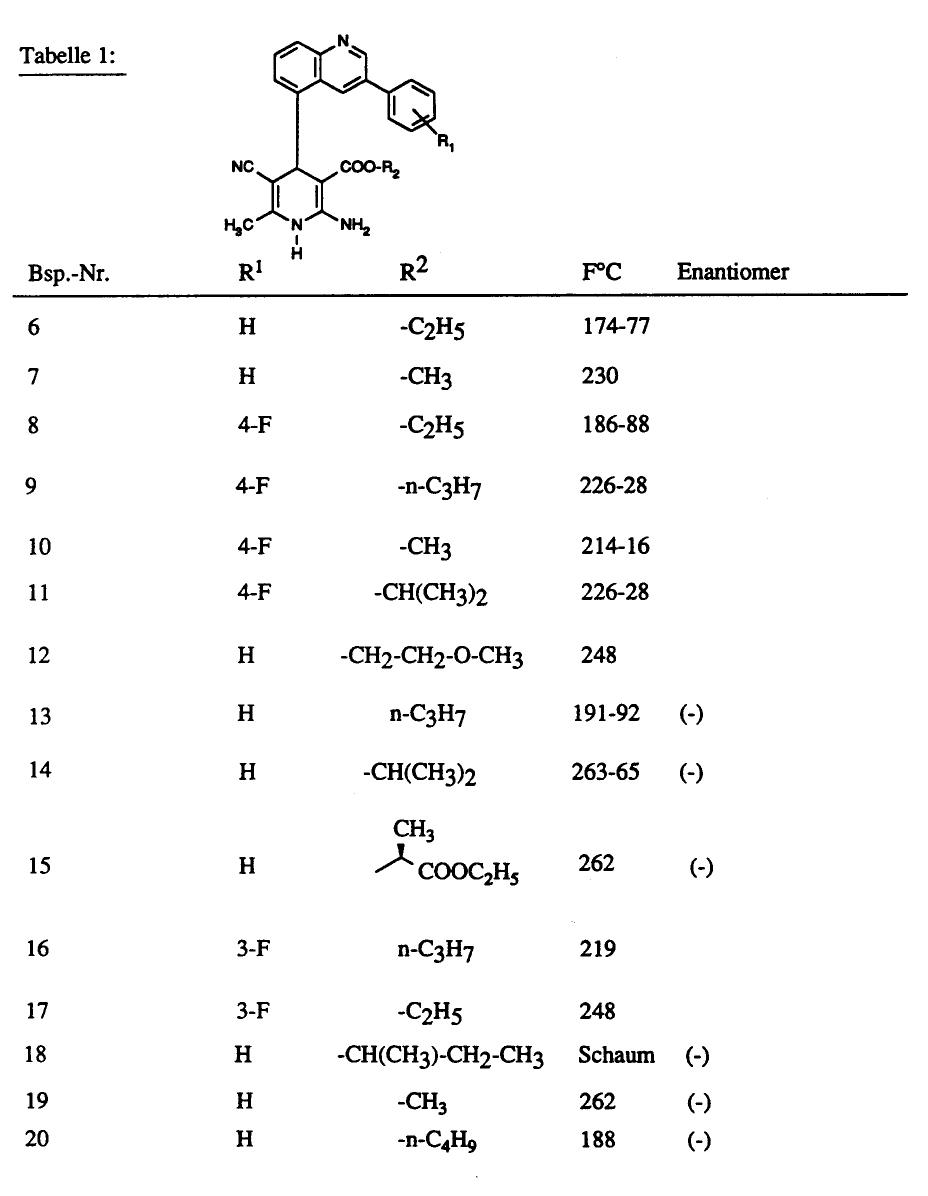

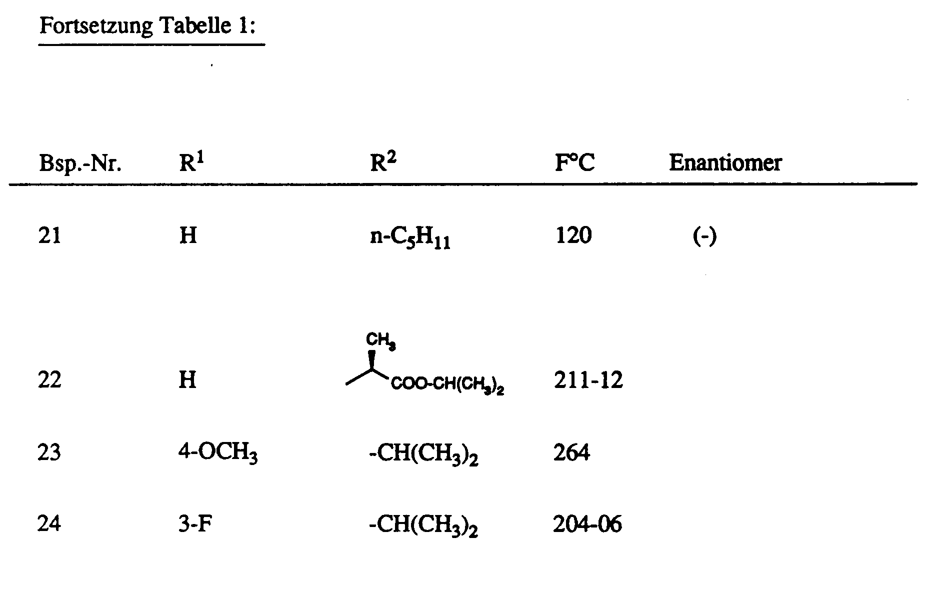

Die in Tabelle 2 aufgeführten Beispiele werden in Analogie zur Vorschrift des Beispiels 1 hergestellt:

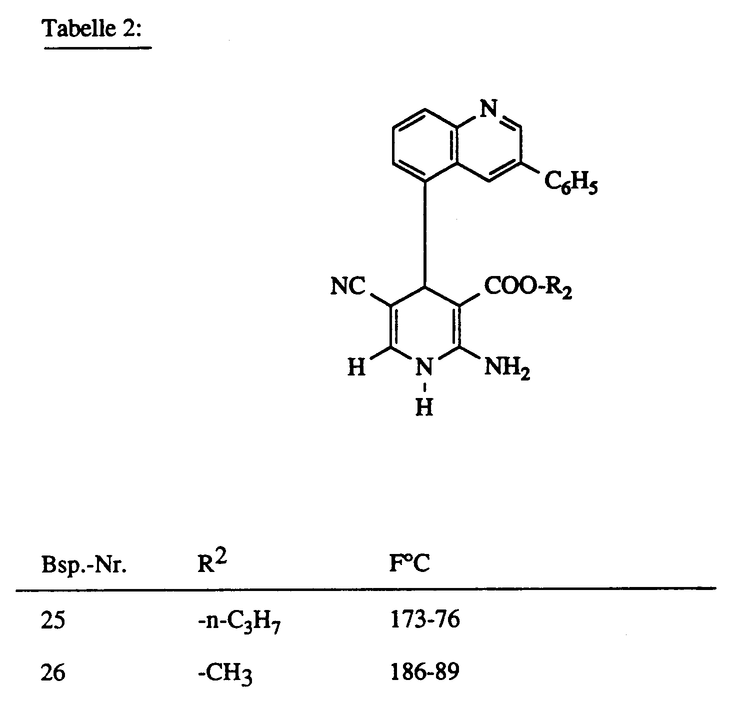

Die in Tabelle 3 aufgeführten Beispiele werden in Analogie zu den Vorschriften der Beispiele 1 bis 5 hergestellt:

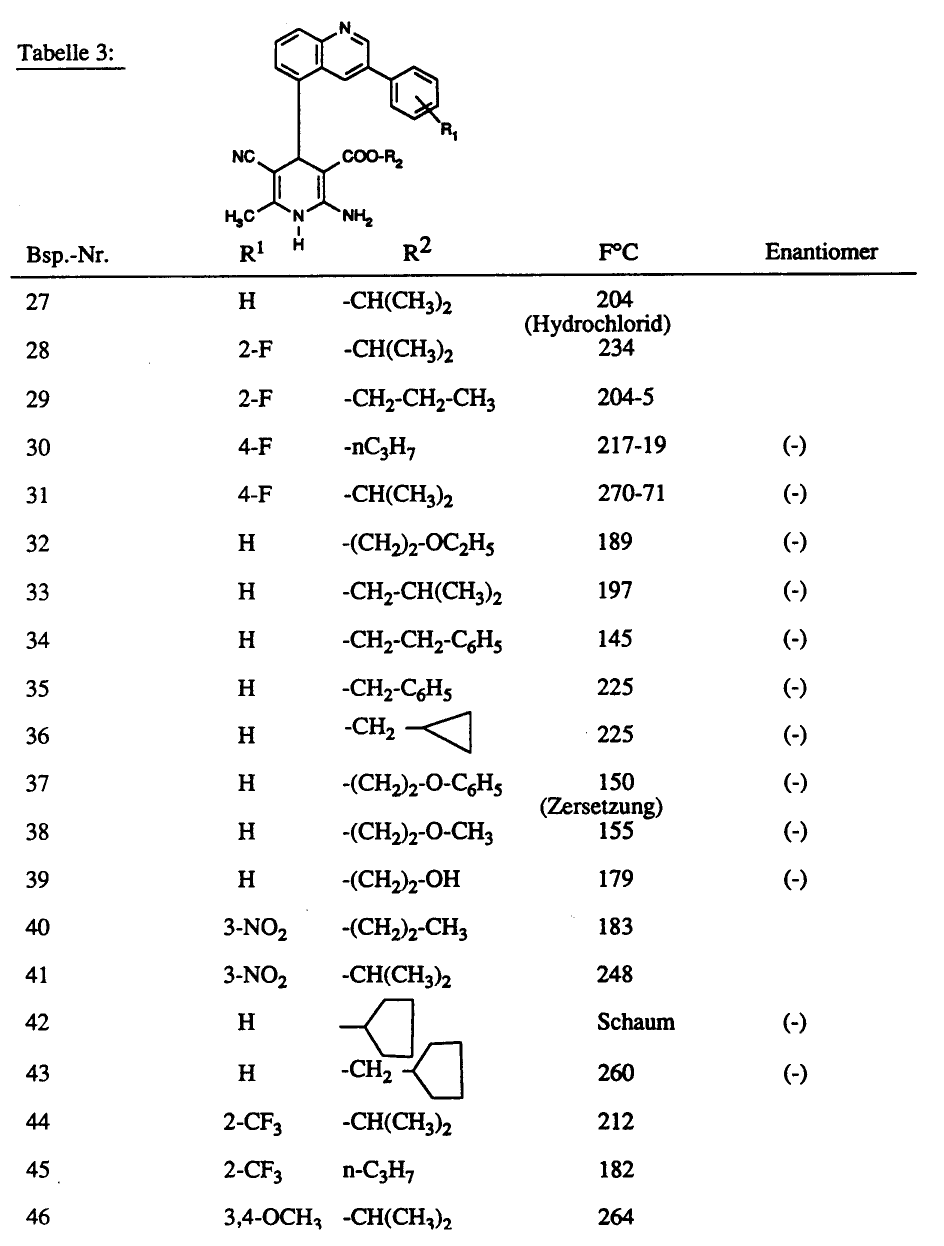

## Patentansprüche (Patentansprüche für folgende(n) Vertragsstaat(en): AT, BE, CH, DE, DK, FR, GB, IT, LI, LU, NL, PT, SE)

1. 2-Amino-5-cyano-4-chinolin-1,4-dihydropyridine der allgemeinen Formel (I) 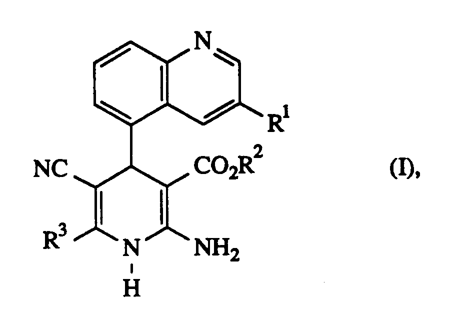 in welcher
R¹ für Aryl mit 6 bis 10 Kohlenstoffatomen steht, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel -NR⁴R⁵ substituiert ist,
worin
R⁴ und R⁵ gleich oder verschieden sind und Wasserstoff, geradkettiges oder verzweigtes Alkyl mit bis zu 8 Kohlenstoffatomen, Phenyl oder Benzyl bedeuten,
oder für Thienyl steht,
R² für Wasserstoff steht, oder
für Cycloalkyl mit 5 bis 8 Kohlenstoffatomen steht oder
für geradkettiges oder verzweigtes Alkyl, Alkenyl, Alkadienyl oder Alkinyl mit jeweils bis zu 10 Kohlenstoffatomen steht, die gegebenenfalls 1- oder 2-fach gleich oder verschieden durch Halogen, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 8 Kohlenstoffatomen, Cycloalkyl mit 3 bis 8 Kohlenstoffatomen oder durch Phenoxy oder Phenyl substituiert sind, wobei die letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können,
oder durch die Gruppe -NR⁴R⁵ substituiert sind,
worin
R⁴ und R⁵ die oben angegebene Bedeutung haben,
R³ für Wasserstoff oder geradkettiges oder verzweigtes Alkyl mit bis zu 8 Kohlenstoffatomen steht,
und deren physiologisch unbedenklichen Salze.

2. Verbindungen der allgemeinen Formel (I) gemäß Anspruch 1, in welcher
R¹ für Phenyl steht, das gegebenenfalls bis zu 3-fach gleich oder verschieden durch Halogen, Nitro, Cyano, Trifluormethyl oder durch geradkettiges oder verzweigtes Alkyl oder Alkoxy mit jeweils bis zu 6 Kohlenstoffatomen, Benzyl oder durch eine Gruppe der Formel -NR⁴R⁵ substituiert ist,
worin
R⁴ und R⁵ gleich oder verschieden sind und Wasserstoff, geradkettiges oder verzweigtes Alkyl mit bis zu 6 Kohlenstoffatomen, Phenyl oder Benzyl bedeuten,
oder für Thienyl steht,
R² für Wasserstoff steht, oder
für Cyclopentyl oder Cyclohexyl steht oder
für geradkettiges oder verzweigtes Alkyl oder Alkenyl mit jeweils bis zu 10 Kohlenstoffatomen steht, die gegebenenfalls durch Halogen, Hydroxy, Carboxy, Cyano oder durch geradkettiges oder verzweigtes Alkylthio, Alkoxycarbonyl, Alkoxy, Acyl oder Acyloxy mit jeweils bis zu 6 Kohlenstoffatomen, Cyclopropyl, Cyclopentyl, Cyclohexyl, Phenoxy oder Phenyl substituiert sind, wobei die beiden letzteren durch Halogen, Methyl, Methoxy oder Ethoxy substituiert sein können,
oder durch die Gruppe -NR⁴R⁵ substituiert sind,
worin
R⁴ und R⁵ die oben angegebene Bedeutung haben,
R³ für Wasserstoff oder geradkettiges oder verzweigtes Alkyl mit bis zu 6 Kohlenstoffatomen steht
und deren physiologisch unbedenklichen Salze.

3. Verbindungen der allgemeinen (I) gemäß Anspruch 1, in welcher
R¹ für Phenyl steht, das gegebenenfalls bis zu 2-fach gleich oder verschieden durch Fluor, Chlor, Nitro, Trifluormethyl oder durch geradkettiges oder verzweigtes Alkyl oder Alkoxy mit jeweils bis zu 4 Kohlenstoffatomen oder durch eine Gruppe der Formel -NR⁴R⁵ substituiert ist,
worin
R⁴ und R⁵ gleich oder verschieden sind und Wasserstoff oder geradkettiges oder verzweigtes Alkyl mit bis zu 4 Kohlenstoffatomen, Phenyl oder Benzyl bedeuten,
oder für Thienyl steht,
R² für Wasserstoff steht, oder
für Cyclopentyl steht oder
für geradkettiges oder verzweigtes Alkyl mit bis zu 8 Kohlenstoffatomen steht, das gegebenenfalls durch Fluor, Chlor, Hydroxy, Carboxy, Cyano oder durch geradkettiges oder verzweigtes Alkoxycarbonyl, Alkoxy oder Acyloxy mit jeweils bis zu 4 Kohlenstoffatomen, Phenyl, Phenoxy, Cyclopropyl, Cyclopentyl oder Cyclohexyl oder durch die Gruppe -NR⁴R⁵ substituiert ist,
worin
R⁴ und R⁵ die oben angegebene Bedeutung haben,
R³ für Wasserstoff oder für geradkettiges oder verzweigtes Alkyl mit bis zu 4 Kohlenstoffatomen steht,
und deren physiologisch unbedenklichen Salze.

4. Verbindungen der allgemeinen Formel (I) gemäß Anspruch 1, zur Verwendung bei der Bekämpfung von Erkrankungen.

5. Verfahren zur Herstellung von Verbindungen der allgemeinen Formel (I) 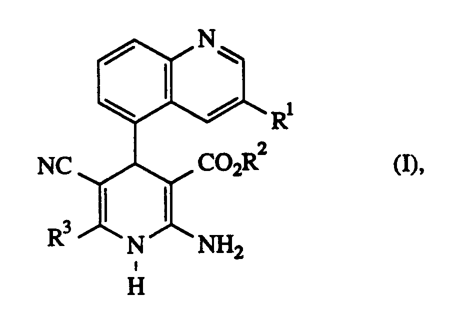 in welcher
R¹ für Aryl mit 6 bis 10 Kohlenstoffatomen steht, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel -NR⁴R⁵ substituiert ist,
worin
R⁴ und R⁵ gleich oder verschieden sind und Wasserstoff, geradkettiges oder verzweigtes Alkyl mit bis zu 8 Kohlenstoffatomen, Phenyl oder Benzyl bedeuten,
oder für Thienyl steht,
R² für Wasserstoff steht, oder
für Cycloalkyl mit 5 bis 8 Kohlenstoffatomen steht oder
für geradkettiges oder verzweigtes Alkyl, Alkenyl, Alkadienyl oder Alkinyl mit jeweils bis zu 10 Kohlenstoffatomen steht, die gegebenenfalls 1- oder 2-fach gleich oder verschieden durch Halogen, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 8 Kohlenstoffatomen, Cycloalkyl mit 3 bis 8 Kohlenstoffatomen oder durch Phenoxy oder Phenyl substituiert sind, wobei die letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können,
oder durch die Gruppe -NR⁴R⁵ substituiert sind,
worin
R⁴ und R⁵ die oben angegebene Bedeutung haben,
R³ für Wasserstoff oder geradkettiges oder verzweigtes Alkyl mit bis zu 8 Kohlenstoffatomen steht,
und deren physiologisch unbedenklichen Salze,
dadurch gekennzeichnet, daß man
[A] entweder Aldehyde der allgemeinen Formel (II) 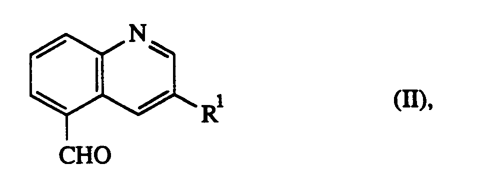 in welcher
R¹ die oben angegebene Bedeutung hat,
direkt mit Verbindungen der allgemeinen Formel (III) 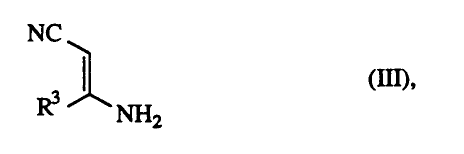 in welcher
R³ die oben angegebene Bedeutung hat,
und Verbindungen der tautomeren Formeln (IV) oder (IVa) 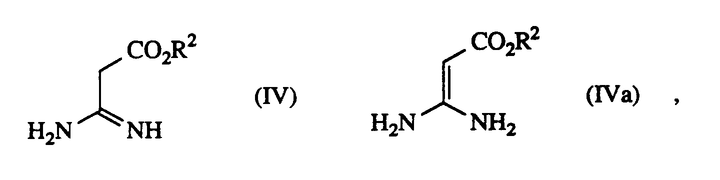 in welcher
R² die oben angegebene Bedeutung hat,
in inerten Lösemittteln bei Temperaturen zwischen 10°C und 150°C umsetzt, oder
[B] Ylidenverbindungen der allgemeinen Formel (V) 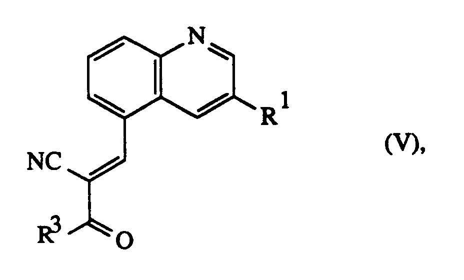 in welcher
R¹ und R³ die oben angegebene Bedeutung haben,
mit Verbindungen der allgemeinen Formel (VI) bzw. (VIa) 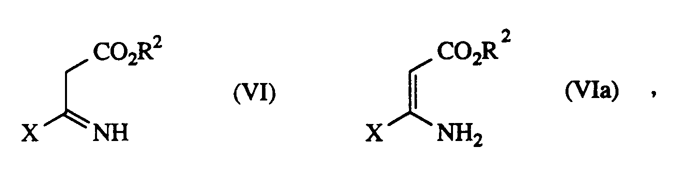 in welcher
R² die oben angegebene Bedeutung hat, und
X für die Aminogruppe oder die Gruppe OR⁶ steht, wobei
R⁶ für C₁-C₄-Akyl steht
gegebenenfalls in Anwesenheit von inerten organischen Lösemitteln bei Temperaturen von 10°C bis 150°C umsetzt, wobei für den Fall, daß X für die Gruppe OR⁶ steht, Ammoniumsalze, wie Ammoniumacetat zugegeben werden.

6. Ylidenverbindungen der allgemeinen Formel (V), 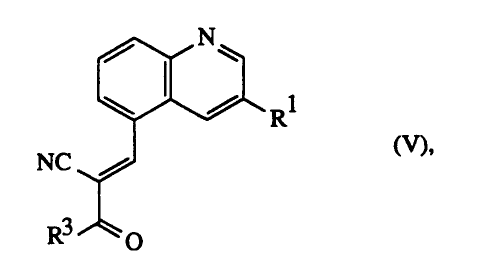 in welcher
R¹ für Aryl mit 6 bis 10 Kohlenstoffatomen steht, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel -NR⁴R⁵ substituiert ist,
worin
R⁴ und R⁵ gleich oder verschieden sind und Wasserstoff, geradkettiges oder verzweigtes Alkyl mit bis zu 8 Kohlenstoffatomen, Phenyl oder Benzyl bedeuten,
oder für Thienyl steht, und
R³ für Wasserstoff oder geradkettiges oder verzweigtes Alkyl mit bis zu 8 Kohlenstoffatomen steht.

7. Verfahren zur Herstellung von Verbindungen der allgemeinen Formel (V) gemäß Anspruch 6, dadurch gekennzeichnet, daß man Verbindungen der allgemeinen Formel (VII) 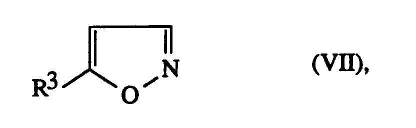 in welcher
R³ die in Anspruch 6 angegebene Bedeutung hat,
mit Alkali-hydroxiden oder -alkoholaten in die Alkalisalze der Verbindungen der allgemeinen Formel (VIII)
R³-CO-CH₂-CN (VIII),
in welcher
R³ die in Anspruch 6 angegebene Bedeutung hat,
gegebenenfalls in Gegenwart von inerten organischen Lösungsmitteln und gegebenenfalls in Gegenwart von Säuren, vorzugsweise Essigsäure, umsetzt und diese mit Aldehyden der allgemeinen Formel (II) 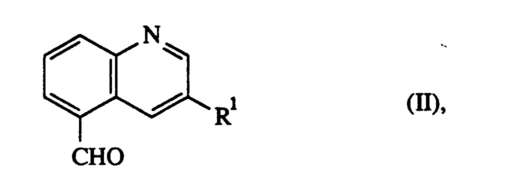 in welcher
R¹ die in Anspruch 6 angegebene Bedeutung hat,
in inerten organischen Lösemitteln unter Zusatz von Säure und gegebenenfalls in Anwesenheit eines Katalysators wie Piperidinacetat bei Temperaturen zwischen 0°C und 150°C umsetzt.

8. Arzneimittel enthaltend mindestens eine Verbindung der allgemeinen Formel (I) gemäß Anspruch 1.

9. Verfahren zur Herstellung von Arzneimitteln, dadurch gekennzeichnet, daß man mindestens eine Verbindung der allgemeinen Formel (I) gemäß Anspruch 1 gegebenenfalls unter Verwendung von üblichen Hilfs- und Trägerstoffen in eine geeignete Applikationsform überführt.

10. Verwendung von Verbindungen der allgemeinen Formel (I) gemäß Anspruch 1 bei der Herstellung von Herz-Kreislauf-wirksamen Arzneimitteln.

## Patentansprüche (Patentansprüche für folgende(n) Vertragsstaat(en): ES, GR)

1. Verfahren zur Herstellung von Verbindungen der allgemeinen Formel (I) 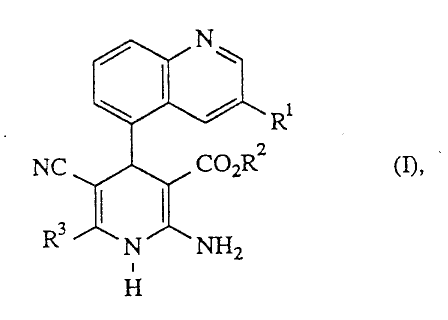 in welcher
R¹ für Aryl mit 6 bis 10 Kohlenstoffatomen steht, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel -NR⁴R⁵ substituiert ist,
worin
R⁴ und R⁵ gleich oder verschieden sind und Wasserstoff, geradkettiges oder verzweigtes Alkyl mit bis zu 8 Kohlenstoffatomen, Phenyl oder Benzyl bedeuten,
oder für Thienyl steht,
R² für Wasserstoff steht, oder
für Cycloalkyl mit 5 bis 8 Kohlenstoffatomen steht oder
für geradkettiges oder verzweigtes Alkyl, Alkenyl, Alkadienyl oder Alkinyl mit jeweils bis zu 10 Kohlenstoffatomen steht, die gegebenenfalls 1- oder 2-fach gleich oder verschieden durch Halogen, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 8 Kohlenstoffatomen, Cycloalkyl mit 3 bis 8 Kohlenstoffatomen oder durch Phenoxy oder Phenyl substituiert sind, wobei die letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können,
oder durch die Gruppe -NR⁴R⁵ substituiert sind,
worin
R⁴ und R⁵ die oben angegebene Bedeutung haben,
R³ für Wasserstoff oder geradkettiges oder verzweigtes Alkyl mit bis zu 8 Kohlenstoffatomen steht,
und deren physiologisch unbedenklichen Salze,
dadurch gekennzeichnet, daß man
[A] entweder Aldehyde der allgemeinen Formel (II) 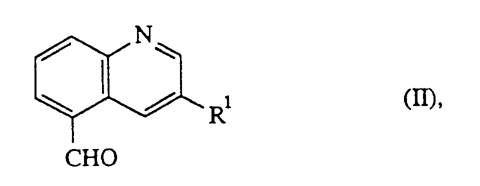 in welcher
R¹ die oben angegebene Bedeutung hat,
direkt mit Verbindungen der allgemeinen Formel (III) 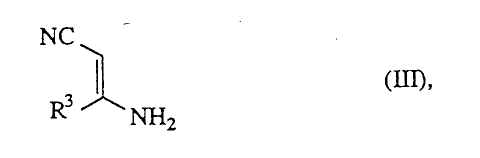 in welcher
R³ die oben angegebene Bedeutung hat,
und Verbindungen der tautomeren Formeln (IV) oder (IVa) 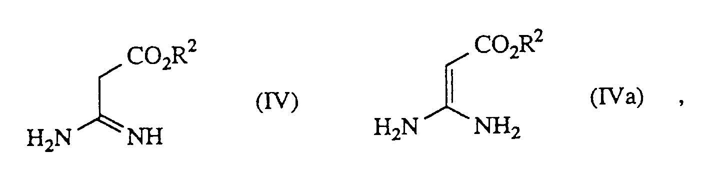 in welcher
R² die oben angegebene Bedeutung hat,
in inerten Lösemittteln bei Temperaturen zwischen 10°C und 150°C umsetzt, oder
[B] Ylidenverbindungen der allgemeinen Formel (V) 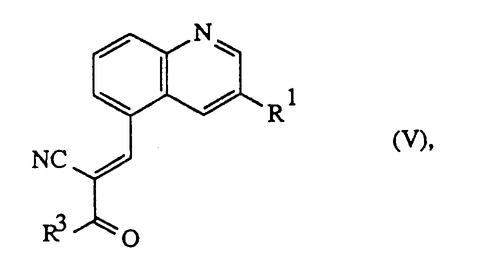 in welcher
R¹ und R³ die oben angegebene Bedeutung haben,
mit Verbindungen der allgemeinen Formel (VI) bzw. (VIa) 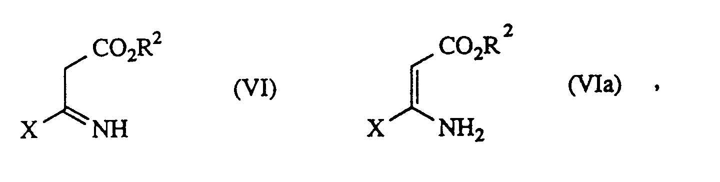 in welcher
R² die oben angegebene Bedeutung hat, und
X für die Aminogruppe oder die Gruppe OR⁶ steht, wobei
R⁶ für C₁-C₄-Akyl steht
gegebenenfalls in Anwesenheit von inerten organischen Lösemitteln bei Temperaturen von 10°C bis 150°C umsetzt, wobei für den Fall, daß X für die Gruppe OR⁶ steht, Ammoniumsalze, wie Ammoniumacetat zugegeben werden.

2. Verfahren zur Herstellung von Verbindungen der allgemeinen Formel (V) 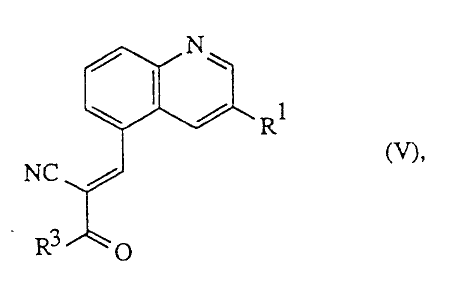 in welcher
R¹ für Aryl mit 6 bis 10 Kohlenstoffatomen steht, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel -NR⁴R⁵ substituiert ist,
worin
R⁴ und R⁵ gleich oder verschieden sind und Wasserstoff, geradkettiges oder verzweigtes Alkyl mit bis zu 8 Kohlenstoffatomen, Phenyl oder Benzyl bedeuten,
oder für Thienyl steht, und
R³ für Wasserstoff oder geradkettiges oder verzweigtes Alkyl mit bis zu 8 Kohlenstoffatomen steht,
dadurch gekennzeichnet, daß man Verbindungen der allgemeinen Formel (VII) 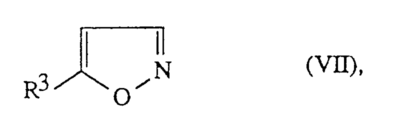 in welcher
R³ die in Anspruch 6 angegebene Bedeutung hat,
mit Alkali-hydroxiden oder -alkoholaten in die Alkalisalze der Verbindungen der allgemeinen Formel (VIII)
R³ - CO - CH₂ - CN (VIII),
in welcher
R³ die in Anspruch 6 angegebene Bedeutung hat,
gegebenenfalls in Gegenwart von inerten organischen Lösungsmitteln und gegebenenfalls in Gegenwart von Säuren, vorzugsweise Essigsäure, umsetzt und diese mit Aldehyden der allgemeinen Formel (II) 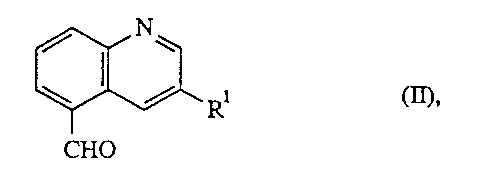 in welcher
R¹ die in Anspruch 6 angegebene Bedeutung hat,
in inerten organischen Lösemitteln unter Zusatz von Säure und gegebenenfalls in Anwesenheit eines Katalysators wie Piperidinacetat bei Temperaturen zwischen 0°C und 150° umsetzt.

3. Verfahren zur Herstellung von Arzneimitteln, dadurch gekennzeichnet, daß man mindestens eine Verbindung der allgemeinen Formel (I) gemäß Anspruch 1 gegebenenfalls unter Verwendung von üblichen Hilfs- und Trägerstoffen in eine geeignete Applikationsform überführt.

## Claims (Claims for the following Contracting State(s): AT, BE, CH, DE, DK, FR, GB, IT, LI, LU, NL, PT, SE)

1. 2-Amino-5-cyano-4-quinoline-1,4-dihydropyridines of the general formula (I) 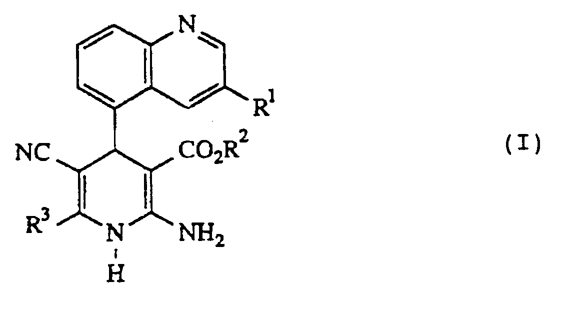 in which
R¹ represents aryl having 6 to 10 carbon atoms, which is optionally substituted up to 3 times, in an identical or different manner, by halogen, nitro, cyano, trifluoromethyl, trifluoromethoxy or trifluoromethylthio,
or by straight-chain or branched alkyl having up to 8 carbon atoms, which can in turn be substituted by aryl having 6 to 10 carbon atoms,
or is substituted by straight-chain or branched alkoxy or alkoxycarbonyl having in each case up to 8 carbon atoms, carboxyl or amino, or by a group of the formula -NR⁴R⁵,
wherein
R⁴ and R⁵ are identical or different and denote straight-chain or branched alkyl having up to 8 carbon atoms, phenyl or benzyl,
or represents thienyl,
R² represents hydrogen, or
represents cycloalkyl having 5 to 8 carbon atoms, or represents straight-chain or branched alkyl, alkenyl, alkadienyl or alkinyl having in each case up to 10 carbon atoms, which are optionally substituted once or twice, in an identical or different manner, by halogen, hydroxyl, carboxyl, cyano or nitro, or by straight-chain or branched alkylthio, alkoxy, alkoxycarbonyl, acyl or acyloxy having in each case up to 8 carbon atoms, or cycloalkyl having 3 to 8 carbon atoms, or by phenoxy or phenyl, it being possible for the latter in turn to be substituted up to twice, in an identical or different manner, by halogen or by straight-chain or branched alkyl or alkoxy having in each case up to 6 carbon atoms,
or are substituted by the group -NR⁴R⁵,
wherein
R⁴ and R⁵ have the abovementioned meaning, and
R³ represents hydrogen or straight-chain or branched alkyl having up to 8 carbon atoms,
and physiologically acceptable salts thereof.

2. Compounds of the general formula (I) according to Claim 1, in which
R¹ represents phenyl, which is optionally substituted up to 3 times, in an identical or different manner, by halogen, nitro, cyano or trifluoromethyl, or by straight-chain or branched alkyl or alkoxy having in each case up to 6 carbon atoms, benzyl or by a group of the formula -NR⁴R⁵,
wherein
R⁴ and R⁵ are identical or different and denote hydrogen, straight-chain or branched alkyl having up to 6 carbon atoms, phenyl or benzyl,
or represents thienyl,
R² represents hydrogen,
or represents cyclopentyl or cyclohexyl, or represents straight-chain or branched alkyl or alkenyl having in each case up to 10 carbon atoms, which are optionally substituted by halogen, hydroxyl, carboxyl or cyano, or by straight-chain or branched alkylthio, alkoxycarbonyl, alkoxy, acyl or acyloxy having in each case up to 6 carbon atoms, cyclopropyl, cyclopentyl, cyclohexyl, phenoxy or phenyl, it being possible for the last two to be substituted by halogen, methyl, methoxy or ethoxy, or are substituted by the group -NR⁴R⁵,
wherein
R⁴ and R⁵ have the abovementioned meaning, and
R³ represents hydrogen or straight-chain or branched alkyl having up to 6 carbon atoms,
and physiologically acceptable salts thereof.

3. Compounds of the general formula (I) according to Claim 1, in which
R¹ represents phenyl, which is optionally substituted up to twice, in an identical or different manner, by fluorine, chlorine, nitro or trifluoromethyl, or by straight-chain or branched alkyl or alkoxy having in each case up to 4 carbon atoms, or by a group of the formula -NR⁴R⁵,
wherein
R⁴ and R⁵ are identical or different and denote hydrogen or straight-chain or branched alkyl having up to 4 carbon atoms, phenyl or benzyl,
or represents thienyl,
R² represents hydrogen, or
represents cyclopentyl, or
represents straight-chain or branched alkyl having up to 8 carbon atoms, which is optionally substituted by fluorine, chlorine, hydroxyl, carboxyl or cyano, or by straight-chain or branched alkoxycarbonyl, alkoxy or acyloxy having in each case up to 4 carbon atoms, phenyl, phenoxy, cyclopropyl, cyclopentyl or cyclohexyl, or by the group -NR⁴R⁵,
wherein
R⁴ and R⁵ have the abovementioned meaning, and
R³ represents hydrogen, or represents straight-chain or branched alkyl having up to 4 carbon atoms,
and physiologically acceptable salts thereof.

4. Compounds of the general formula (I) according to Claim 1, for use in combating diseases.

5. Process for the preparation of compounds of the general formula (I) 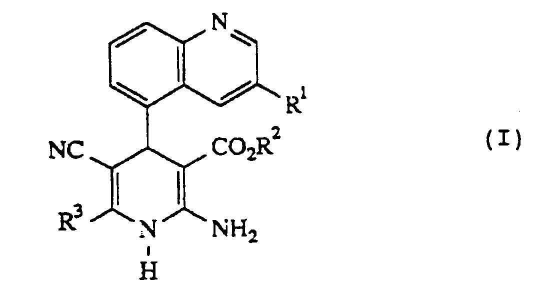 in which
R¹ represents aryl having 6 to 10 carbon atoms, which is optionally substituted up to 3 times, in an identical or different manner, by halogen, nitro, cyano, trifluoromethyl, trifluoromethoxy or trifluoromethylthio,
or by straight-chain or branched alkyl having up to 8 carbon atoms, which can in turn be substituted by aryl having 6 to 10 carbon atoms,
or is substituted by straight-chain or branched alkoxy or alkoxycarbonyl having in each case up to 8 carbon atoms, carboxyl or amino, or by a group of the formula -NR⁴R⁵,
wherein
R⁴ and R⁵ are identical or different and denote straight-chain or branched alkyl having up to 8 carbon atoms, phenyl or benzyl,
or represents thienyl,
R² represents hydrogen, or
represents cycloalkyl having 5 to 8 carbon atoms, or represents straight-chain or branched alkyl, alkenyl, alkadienyl or alkinyl having in each case up to 10 carbon atoms, which are optionally substituted once or twice, in an identical or different manner, by halogen, hydroxyl, carboxyl, cyano or nitro, or by straight-chain or branched alkylthio, alkoxy, alkoxycarbonyl, acyl or acyloxy having in each case up to 8 carbon atoms, or cycloalkyl having 3 to 8 carbon atoms, or by phenoxy or phenyl, it being possible for the latter in turn to be substituted up to twice, in an identical or different manner, by halogen or by straight-chain or branched alkyl or alkoxy having in each case up to 6 carbon atoms,
or are substituted by the group -NR⁴R⁵,
wherein
R⁴ and R⁵ have the abovementioned meaning, and
R³ represents hydrogen or straight-chain or branched alkyl having up to 8 carbon atoms,
and physiologically acceptable salts thereof,
characterised in that
[A] either aldehydes of the general formula (II) 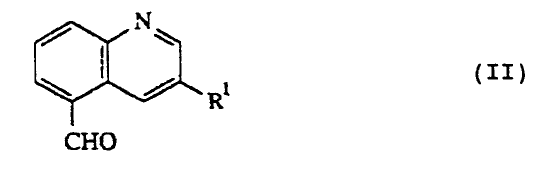 in which
R¹ has the abovementioned meaning,
are reacted directly with compounds of the general formula (III) 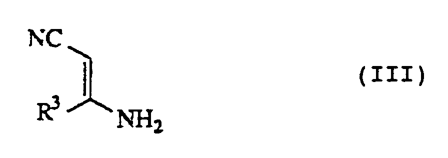 in which
R³ has the abovementioned meaning,
and compounds of the tautomeric formulae (IV) or (IVa) 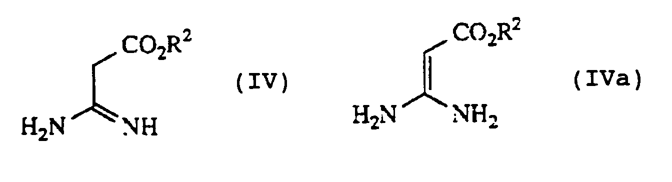 in which
R² has the abovementioned meaning,
in inert solvents at temperatures between 10°C and 150°C,
[B] or ylidene compounds of the general formula (V) 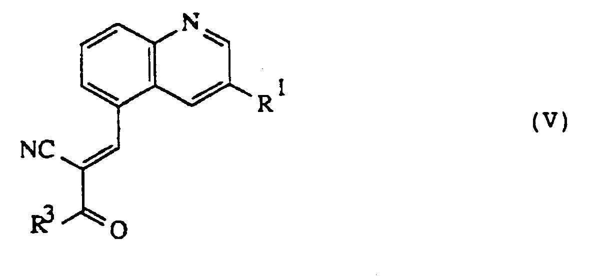 in which 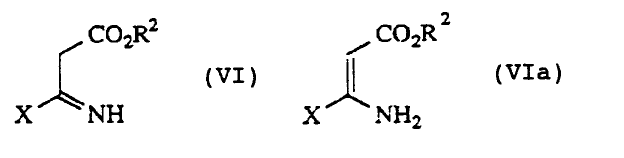 R¹ and R³ have the abovementioned meaning,
are reacted with compounds of the general formula (VI) or (VIa) 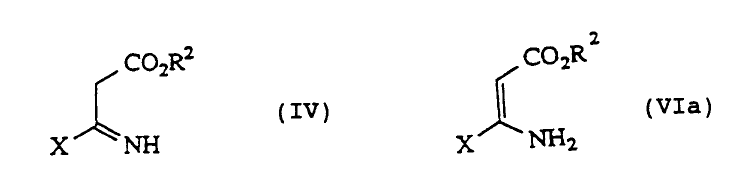 in which
R² has the abovementioned meaning and
X represents the amino group or the group OR⁶, wherein
R⁶ represents C₁-C₄-alkyl,
if appropriate in the presence of inert organic solvents at temperatures of 10°C to 150°C, ammonium salts, such as ammonium acetate being added in the case where X represents the group OR⁶.

6. Ylidene compounds of the general formula (V) 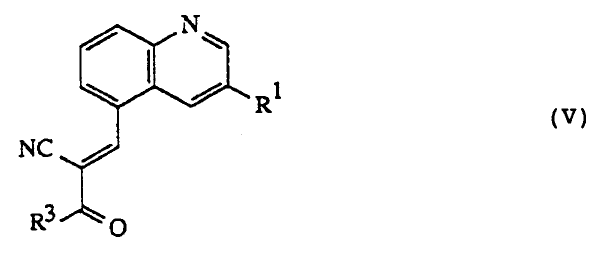 in which
R¹ represents aryl having 6 to 10 carbon atoms, which is optionally substituted up to 3 times, in an identical or different manner, by halogen, nitro, cyano, trifluoromethyl, trifluoromethoxy or trifluoromethylthio,
or by straight-chain or branched alkyl having up to 8 carbon atoms, which can in turn be substituted by aryl having 6 to 10 carbon atoms,
or is substituted by straight-chain or branched alkoxy or alkoxycarbonyl having in each case up to 8 carbon atoms, carboxyl or amino, or by a group of the formula -NR⁴R⁵,
wherein
R⁴ and R⁵ are identical or different and denote straight-chain or branched alkyl having up to 8 carbon atoms, phenyl or benzyl,
or represents thienyl, and
R³ represents hydrogen or straight-chain or branched alkyl having up to 8 carbon atoms.

7. Process for the preparation of compounds of the general formula (V) according to Claim 6, characterised in that compounds of the general formula (VII) 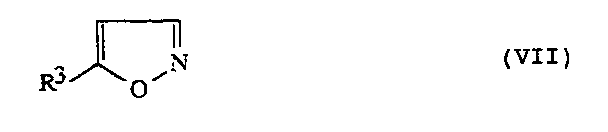 in which
R³ has the meaning given in Claim 6,
are converted with alkali metal hydroxides or alcoholates into the alkali metal salts of the compounds of the general formula (VIII)
R³ - CO - CH₂ - CN (VIII)
in which
R³ has the meaning given in Claim 6,
if appropriate in the presence of inert organic solvents and if appropriate in the presence of acids, preferably acetic acid, and these are reacted with aldehydes of the general formula (II) 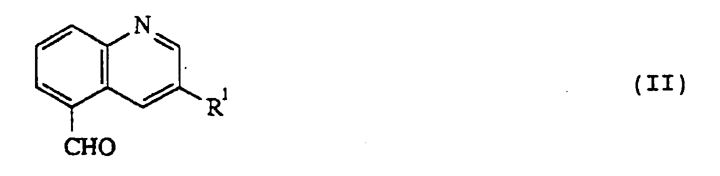 in which
R¹ has the meaning given in Claim 6,
in inert organic solvents, with the addition of acid and if appropriate in the presence of a catalyst, such as piperidine acetate, at temperatures between 0°C and 150°C.

8. Medicaments containing at least one compound of the general formula (I) according to Claim 1.

9. Process for the preparation of medicaments, characterised in that at least one compound of the general formula (I) according to Claim 1 is converted into a suitable administration form, if appropriate using customary auxiliaries and excipients.

10. Use of compounds of the general formula (I) according to Claim 1 in the preparation of medicaments having a cardiovascular action.

## Claims (Claims for the following Contracting State(s): ES, GR)

1. Process for the preparation of compounds of the general formula (I) 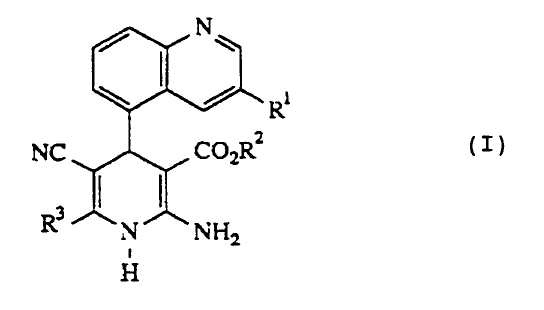 in which
R¹ represents aryl having 6 to 10 carbon atoms, which is optionally substituted up to 3 times, in an identical or different manner, by halogen, nitro, cyano, trifluoromethyl, trifluoromethoxy or trifluoromethylthio,
or by straight-chain or branched alkyl having up to 8 carbon atoms, which can in turn be substituted by aryl having 6 to 10 carbon atoms,
or is substituted by straight-chain or branched alkoxy or alkoxycarbonyl having in each case up to 8 carbon atoms, carboxyl or amino, or by a group of the formula -NR⁴R⁵,
wherein
R⁴ and R⁵ are identical or different and denote straight-chain or branched alkyl having up to 8 carbon atoms, phenyl or benzyl,
or represents thienyl,
R² represents hydrogen, or
represents cycloalkyl having 5 to 8 carbon atoms, or represents straight-chain or branched alkyl, alkenyl, alkadienyl or alkinyl having in each case up to 10 carbon atoms, which are optionally substituted once or twice, in an identical or different manner, by halogen, hydroxyl, carboxyl, cyano or nitro, or by straight-chain or branched alkylthio, alkoxy, alkoxycarbonyl, acyl or acyloxy having in each case up to 8 carbon atoms, or cycloalkyl having 3 to 8 carbon atoms, or by phenoxy or phenyl, it being possible for the latter in turn to be substituted up to twice, in an identical or different manner, by halogen or by straight-chain or branched alkyl or alkoxy having in each case up to 6 carbon atoms,
or are substituted by the group -NR⁴R⁵,
wherein
R⁴ and R⁵ have the abovementioned meaning, and
R³ represents hydrogen or straight-chain or branched alkyl having up to 8 carbon atoms,
and physiologically acceptable salts thereof,
characterised in that
[A] either aldehydes of the general formula (II) 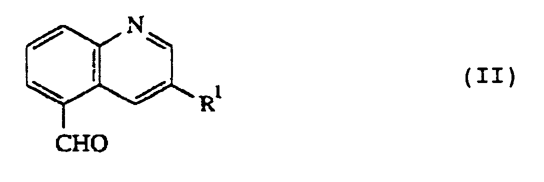 in which
R¹ has the abovementioned meaning,
are reacted directly with compounds of the general formula (III) 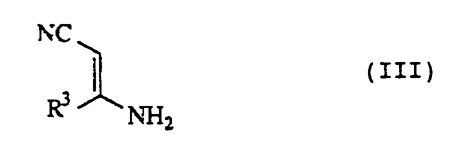 in which
R³ has the abovementioned meaning,
and compounds of the tautomeric formulae (IV) or (IVa) 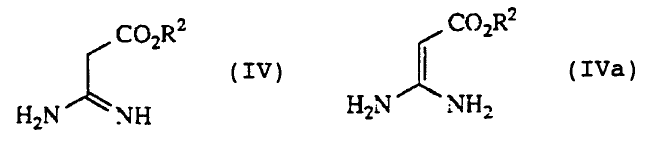 in which
R² has the abovementioned meaning,
in inert solvents at temperatures between 10°C and 150°C,
[B] or ylidene compounds of the general formula (V) 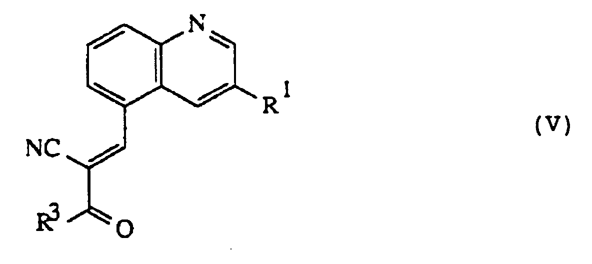 in which 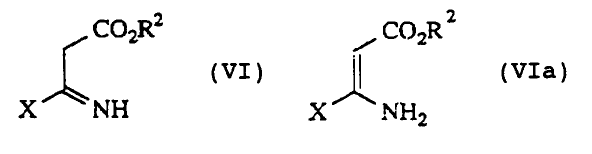 R¹ and R³ have the abovementioned meaning,
are reacted with compounds of the general formula (VI) or (VIa) 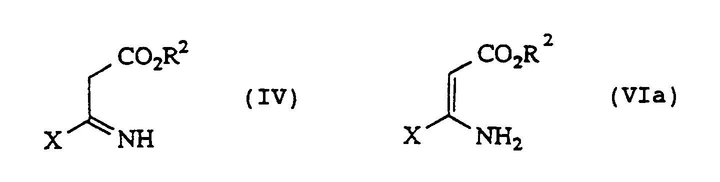 in which
R² has the abovementioned meaning and
X represents the amino group or the group OR⁶, wherein
R⁶ represents C₁-C₄-alkyl,
if appropriate in the presence of inert organic solvents at temperatures of 10°C to 150°C, ammonium salts, such as ammonium acetate being added in the case where X represents the group OR⁶.

2. Process for the preparation of compounds of the general formula (V) 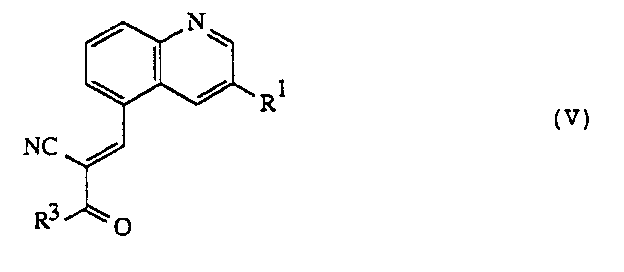 in which
R¹ represents aryl having 6 to 10 carbon atoms, which is optionally substituted up to 3 times, in an identical or different manner, by halogen, nitro, cyano, trifluoromethyl, trifluoromethoxy or trifluoromethylthio,
or by straight-chain or branched alkyl having up to 8 carbon atoms, which can in turn be substituted by aryl having 6 to 10 carbon atoms,
or is substituted by straight-chain or branched alkoxy or alkoxycarbonyl having in each case up to 8 carbon atoms, carboxyl or amino, or by a group of the formula -NR⁴R⁵,
wherein
R⁴ and R⁵ are identical or different and denote straight-chain or branched alkyl having up to 8 carbon atoms, phenyl or benzyl,
or represents thienyl, and
R³ represents hydrogen or straight-chain or branched alkyl having up to 8 carbon atoms, characterised in that compounds of the general formula (VII) 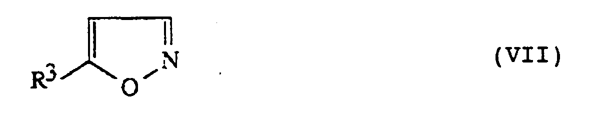
in which
R³ has the meaning given in Claim 6,
are converted with alkali metal hydroxides or alcoholates into the alkali metal salts of the compounds of the general formula (VIII)
R³ - CO - CH₂ - CN (VIII)
in which
R³ has the meaning given in Claim 6,
if appropriate in the presence of inert organic solvents and if appropriate in the presence of acids, preferably acetic acid, and these are reacted with aldehydes of the general formula (II) 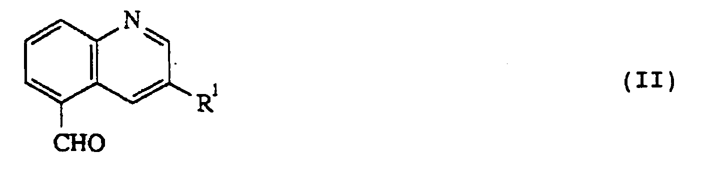 in which
R¹ has the meaning given in Claim 6,
in inert organic solvents, with the addition of acid and if appropriate in the presence of a catalyst, such as piperidine acetate, at temperatures between 0°C and 150°C.

3. Process for the preparation of medicaments, characterised in that at least one compound of the general formula (I) according to Claim 1 is converted into a suitable administration form, if appropriate using customary auxiliaries and excipients.

## Revendications (Revendications pour l'(les) Etat(s) contractant(s) suivant(s): AT, BE, CH, DE, DK, FR, GB, IT, LI, LU, NL, PT, SE)

1. 2-amino-5-cyano-4-quinoléine-1,4-dihydropyridines de formule générale (I) 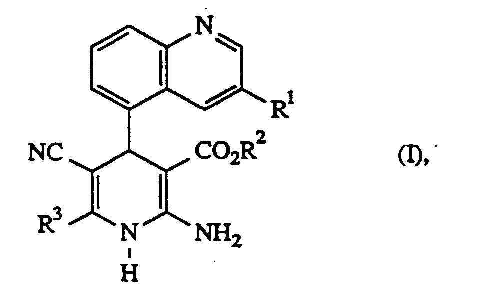 dans laquelle
R¹ est un groupe aryle ayant 6 à 10 atomes de carbone, qui est substitué, le cas échéant, jusqu'à 3 fois, identiques ou différentes, par un halogène, un radical nitro, cyano, trifluorométhyle, trifluorométhoxy, trifluorométhylthio,
ou par un radical alkyle linéaire ou ramifié ayant jusqu'à 8 atomes de carbone, qui peut être substitué de son côté par un radical aryle ayant 6 à 10 atomes de carbone,
ou substitué par un radical alkoxy ou alkoxycarbonyle linéaire ou ramifié ayant chacun jusqu'à 8 atomes de carbone, carboxy, amino ou par un groupe de formule -NR⁴R⁵,
dans laquelle
R⁴ et R⁵ sont identiques ou différents et représentent de l'hydrogène, un radical alkyle linéaire ou ramifié ayant jusqu'à 8 atomes de carbone, phényle ou benzyle,
ou un groupe thiényle,
R² représente de l'hydrogène ou
un groupe cycloalkyle ayant 5 à 8 atomes de carbone ou
un groupe alkyle, alcényle, alcadiényle ou alcynyle linéaire ou ramifié ayant chacun jusqu'à 10 atomes de carbone, ces groupes étant substitués, le cas échéant, 1 ou 2 fois identiques ou différentes, par un radical halogéno, hydroxy, carboxy, cyano, nitro ou par un radical alkylthio, alkoxy, alkoxycarbonyle, acyle ou acyloxy linéaire ou ramifié ayant chacun jusqu'à 8 atomes de carbone, cycloalkyle ayant 3 à 8 atomes de carbone ou par un radical phénoxy ou phényle, ces deux derniers pouvant être substitués quant à eux jusqu'à 2 fois identiques ou différentes par un halogène ou par un radical alkyle ou alkoxy linéaire ou ramifié ayant chacun jusqu'à 6 atomes de carbone,
ou substitués par le groupe -NR⁴R⁵,
dans lequel
R⁴ et R⁵ ont la définition indiquée ci-dessus,
R³ représente de l'hydrogène ou un groupe alkyle linéaire ou ramifié ayant jusqu'à 8 atomes de carbone,
et leurs sels acceptables du point de vue physiologique.

2. Composés de formule générale (I) suivant la revendication 1, dans laquelle
R¹ est un groupe phényle qui est substitué, le cas échéant, jusqu'à 3 fois identiques ou différentes par un halogène, un radical nitro, cyano, trifluorométhyle ou par un radical alkyle ou alkoxy linéaire ou ramifié ayant chacun jusqu'à 6 atomes de carbone, par un radical benzyle ou par un groupe de formule -NR⁴R⁵,
dans laquelle
R⁴ et R⁵ sont identiques ou différents et représentent de l'hydrogène, un radical alkyle linéaire ou ramifié ayant jusqu'à 6 atomes de carbone, phényle ou benzyle,
ou un groupe thiényle,
R² représente de l'hydrogène, ou
un groupe cyclopentyle ou cyclohexyle ou
un groupe alkyle ou alcényle linéaire ou ramifié ayant chacun jusqu'à 10 atomes de carbone, ces groupes étant substitués, le cas échéant par un halogène, un radical hydroxy, carboxy, cyano ou par un radical alkylthio, alkoxycarbonyle, alkoxy, acyle ou acyloxy linéaire ou ramifié ayant chacun jusqu'à 6 atomes de carbone, cyclopropyle, cyclopentyle, cyclohexyle, phénoxy ou phényle, ces deux derniers pouvant être substitués par un radical halogéno, méthyle, méthoxy ou éthoxy,
ou substitués par le groupe -NR⁴R⁵,
dans lequel
R⁴ et R⁵ ont la définition indiquée ci-dessus,
R³ représente de l'hydrogène ou un groupe alkyle linéaire ou ramifié ayant jusqu'à 6 atomes de carbone,
et leurs sels acceptables du point de vue physiologique.

3. Composés de formule générale (I) suivant la revendication 1, dans laquelle
R¹ représente un groupe phényle qui est substitué, le cas échéant, jusqu'à 2 fois identiques ou différentes par du fluor, du chlore, un radical nitro, trifluorométhyle ou par un radical alkyle ou alkoxy linéaire ou ramifié ayant chacun jusqu'à 4 atomes de carbone ou par un groupe de formule -NR⁴R⁵,
dans laquelle
R⁴ et R⁵ sont identiques ou différents et représentent de l'hydrogène ou un radical alkyle linéaire ou ramifié ayant jusqu'à 4 atomes de carbone, phényle ou benzyle,
ou un groupe thiényle,
R² représente de l'hydrogène ou
un groupe cyclopentyle ou
un groupe alkyle linéaire ou ramifié ayant jusqu'à 8 atomes de carbone, qui est substitué le cas échéant par du fluor, du chlore, un radical hydroxy, carboxy, cyano ou par un radical alkoxycarbonyle, alkoxy ou acyloxy linéaire ou ramifié ayant dans chaque cas jusqu'à 4 atomes de carbone, un radical phényle, phénoxy, cyclopropyle, cyclopentyle ou cyclohexyle ou par le groupe -NR⁴R⁵,
où
R⁴ et R⁵ ont la définition indiquée ci-dessus,
R³ représente de l'hydrogène ou un groupe alkyle linéaire ou ramifié ayant jusqu'à 4 atomes de carbone,
et leurs sels acceptables du point de vue physiologique.

4. Composés de formule générale (I) suivant la revendication 1, destinés à être utilisés pour combattre des maladies.

5. Procédé de production de composés de formule générale (I) 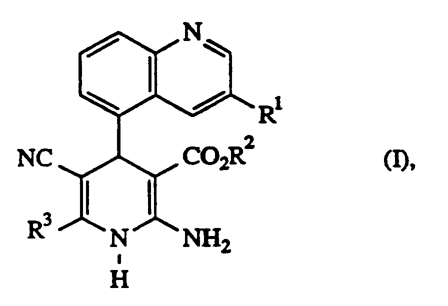 dans laquelle
R¹ est un groupe aryle ayant 6 à 10 atomes de carbone, qui est substitué, le cas échéant, jusqu'à 3 fois, identiques ou différentes, par un halogène, un radical nitro, cyano, trifluorométhyle, trifluorométhoxy, trifluorométhylthio, ou par un radical alkyle linéaire ou ramifié ayant jusqu'à 8 atomes de carbone, qui peut être substitué de son côté par un radical aryle ayant 6 à 10 atomes de carbone,
ou substitué par un radical alkoxy ou alkoxycarbonyle linéaire ou ramifié ayant chacun jusqu'à 8 atomes de carbone, carboxy, amino ou par un groupe de formule -NR⁴R⁵,
dans laquelle
R⁴ et R⁵ sont identiques ou différents et représentent de l'hydrogène, un radical alkyle linéaire ou ramifié ayant jusqu'à 8 atomes de carbone, phényle ou benzyle,
ou un groupe thiényle,
R² représente de l'hydrogène ou
un groupe cycloalkyle ayant 5 à 8 atomes de carbone ou
un groupe alkyle, alcényle, alcadiényle ou alcynyle linéaire ou ramifié ayant chacun jusqu'à 10 atomes de carbone, ces groupes étant substitués, le cas échéant, 1 ou 2 fois identiques ou différentes, par un radical halogéno, hydroxy, carboxy, cyano, nitro ou par un radical alkylthio, alkoxy, alkoxycarbonyle, acyle ou acyloxy linéaire ou ramifié ayant chacun jusqu'à 8 atomes de carbone, cycloalkyle ayant 3 à 8 atomes de carbone ou par un radical phénoxy ou phényle, ces deux derniers pouvant être substitués quant à eux jusqu'à 2 fois identiques ou différentes par un halogène ou par un radical alkyle ou alkoxy linéaire ou ramifié ayant chacun jusqu'à 6 atomes de carbone,
ou substitués par le groupe -NR⁴R⁵,
dans lequel
R⁴ et R⁵ ont la définition indiquée ci-dessus,
R³ représente de l'hydrogène ou un groupe alkyle linéaire ou ramifié ayant jusqu'à 8 atomes de carbone,
et leurs sels acceptables du point de vue physiologique,
caractérisé en ce que
[A] on fait réagir des aldéhydes de formule générale (II) 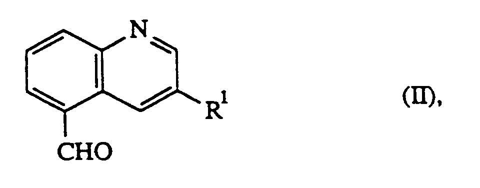 dans laquelle
R¹ a la définition indiquée ci-dessus,
directement avec des composés de formule générale (III) 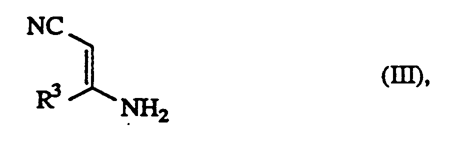 dans laquelle
R³ a la définition indiquée ci-dessus,
et des composés de formules tautomères (IV) ou (IVa) 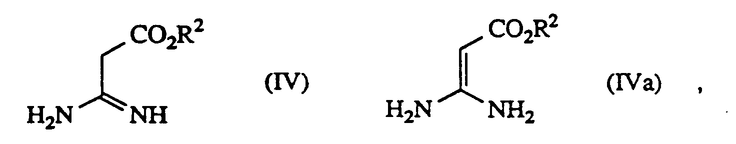 dans lesquelles
R² a la définition indiquée ci-dessus,
dans des solvants inertes à des températures comprises entre 10°C et 150°C,
ou bien
[B] on fait réagir des composés ylidéniques de formule générale (V) 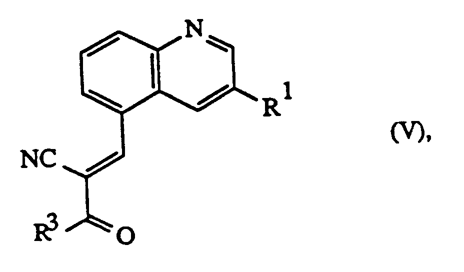 dans laquelle
R¹ et R³ ont la définition indiquée ci-dessus,
avec des composés de formule générale (VI) ou (VIa) 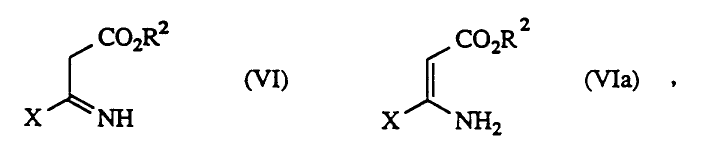 dans laquelle
R² a la définition indiquée ci-dessus, et
X représente le groupe amino ou le groupe OR⁶, dans lequel R⁶ est un radical alkyle en C₁ à C₄,
le cas échéant en présence de solvants organiques inertes à des températures de 10°C à 150°C, et au cas où X représente le groupe OR⁶, on ajoute des sels d'ammonium tels que l'acétate d'ammonium.

6. Composés ylidéniques de formule (V) 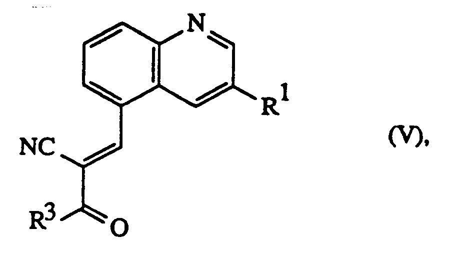 dans laquelle
R¹ est un groupe aryle ayant 6 à 10 atomes de carbone, qui est substitué, le cas échéant jusqu'à 3 fois identiques ou différentes, par un radical halogéno, nitro, cyano, trifluorométhyle, trifluorométhoxy, trifluorométhylthio,
ou par un radical alkyle linéaire ou ramifié ayant jusqu'à 8 atomes de carbone, qui peut être substitué de son côté par un radical aryle ayant 6 à 10 atomes de carbone,
ou substitué par un radical alkoxy ou alkoxycarbonyle linéaire ou ramifié ayant chacun jusqu'à 8 atomes de carbone, carboxy, amino, ou par un groupe de formule -NR⁴R⁵,
dans laquelle
R⁴ et R⁵ sont identiques ou différents et représentent de l'hydrogène, un radical alkyle linéaire ou ramifié ayant jusqu'à 8 atomes de carbone, phényle ou benzyle,
ou un radical thiényle, et
R³ représente de l'hydrogène ou un groupe alkyle linéaire ou ramifié ayant jusqu'à 8 atomes de carbone.

7. Procédé de production de composés de formule générale (V) suivant la revendication 6, caractérisé en ce qu'on fait réagir des composés de formule générale (VII) 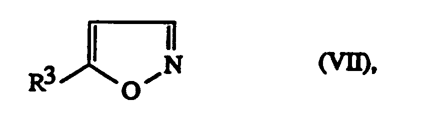 dans laquelle
R³ a la définition indiquée dans la revendication 6,
avec des hydroxydes ou alcoolates alcalins pour former les sels alcalins des composés de formule générale (VIII)
R³-CO-CH₂-CN (VIII)
dans laquelle
R³ a la définition indiquée dans la revendication 6,
le cas échéant en présence de solvants organiques inertes et en la présence éventuelle d'acides, de préférence d'acide acétique, et on fait réagir ces sels avec des aldéhydes de formule générale (II) 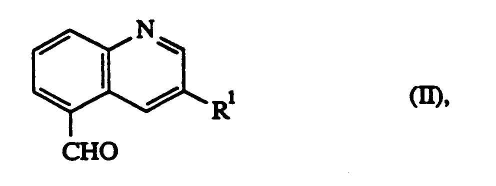 dans laquelle
R¹ a la définition indiquée dans la revendication 6,
à des températures comprises entre 0°C et 150°C dans des solvants organiques inertes avec addition d'acide et, le cas échéant en présence d'un catalyseur tel que l'acétate de pipéridine.

8. Médicament contenant au moins un composé de formule générale (I) suivant la revendication 1.

9. Procédé de production de médicaments, caractérisé en ce qu'on fait prendre une forme d'administration appropriée à au moins un composé de formule générale (I) suivant la revendication 1, le cas échéant en utilisant des substances auxiliaires et des supports classiques.

10. Utilisation de composés de formule générale (I) suivant la revendication 1 pour la préparation de médicaments agissant sur le système cardiovasculaire.

## Revendications (Revendications pour l'(les) Etat(s) contractant(s) suivant(s): ES, GR)

1. Procédé de production de composés de formule générale (I) 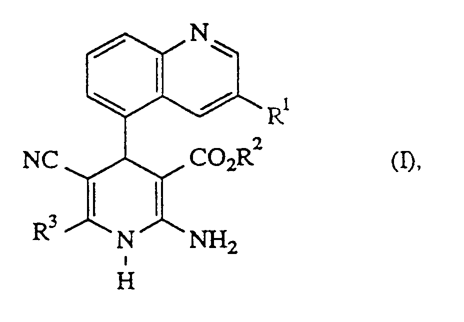 dans laquelle
R¹ est un groupe aryle ayant 6 à 10 atomes de carbone, qui est substitué, le cas échéant, jusqu'à 3 fois, identiques ou différentes, par un halogène, un radical nitro, cyano, trifluorométhyle, trifluorométhoxy, trifluorométhylthio,
ou par un radical alkyle linéaire ou ramifié ayant jusqu'à 8 atomes de carbone, qui peut être substitué de son côté par un radical aryle ayant 6 à 10 atomes de carbone,
ou substitué par un radical alkoxy ou alkoxycarbonyle linéaire ou ramifié ayant chacun jusqu'à 8 atomes de carbone, carboxy, amino ou par un groupe de formule -NR⁴R⁵,
dans laquelle
R⁴ et R⁵ sont identiques ou différents et représentent de l'hydrogène, un radical alkyle linéaire ou ramifié ayant jusqu'à 8 atomes de carbone, phényle ou benzyle,
ou un groupe thiényle,
R² représente de l'hydrogène ou
un groupe cycloalkyle ayant 5 à 8 atomes de carbone ou
un groupe alkyle, alcényle, alcadiényle ou alcynyle linéaire ou ramifié ayant chacun jusqu'à 10 atomes de carbone, ces groupes étant substitués, le cas échéant, 1 ou 2 fois identiques ou différentes, par un radical halogéno, hydroxy, carboxy, cyano, nitro ou par un radical alkylthio, alkoxy, alkoxycarbonyle, acyle ou acyloxy linéaire ou ramifié ayant chacun jusqu'à 8 atomes de carbone, cycloalkyle ayant 3 à 8 atomes de carbone ou par un radical phénoxy ou phényle, ces deux derniers pouvant être substitués quant à eux jusqu'à 2 fois identiques ou différentes par un halogène ou par un radical alkyle ou alkoxy linéaire ou ramifié ayant chacun jusqu'à 6 atomes de carbone,
ou substitués par le groupe -NR⁴R⁵,
dans lequel
R⁴ et R⁵ ont la définition indiquée ci-dessus,
R³ représente de l'hydrogène ou un groupe alkyle linéaire ou ramifié ayant jusqu'à 8 atomes de carbone,
et leurs sels acceptables du point de vue physiologique,
caractérisé en ce que
[A] on fait réagir des aldéhydes de formule générale (II) 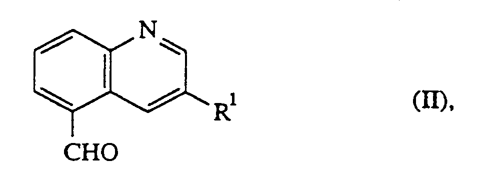 dans laquelle
R¹ a la définition indiquée ci-dessus,
directement avec des composés de formule générale (III) 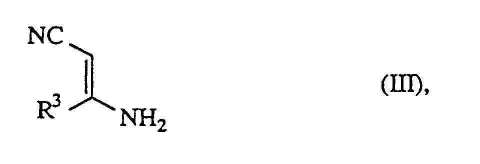 dans laquelle
R³ a la définition indiquée ci-dessus,
et des composés de formules tautomères (IV) ou (IVa) 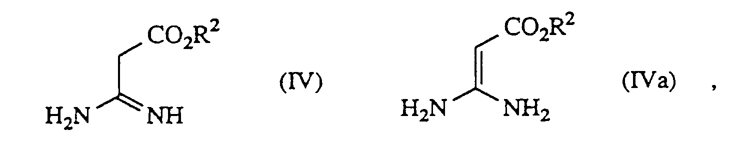 dans lesquelles
R² a la définition indiquée ci-dessus,
dans des solvants inertes à des températures comprises entre 10°C et 150°C,
ou bien
[B] on fait réagir des composés ylidéniques de formule générale (V) 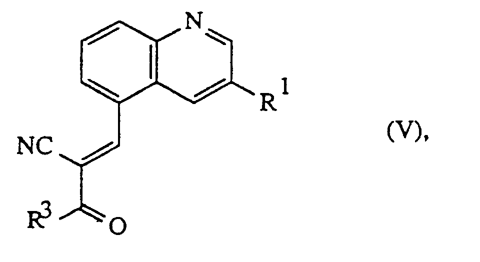 dans laquelle
R¹ et R³ ont la définition indiquée ci-dessus,
avec des composés de formule générale (VI) ou (VIa) 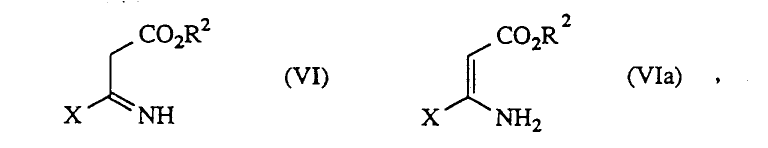 dans laquelle
R² a la définition indiquée ci-dessus, et
X représente le groupe amino ou le groupe OR⁶, dans lequel R⁶ est un radical alkyle en C₁ à C₄,
le cas échéant en présence de solvants organiques inertes à des températures de 10°C à 150°C, et au cas où X représente le groupe OR⁶, on ajoute des sels d'ammonium tels que l'acétate d'ammonium.

2. Procédé de production de composés de formule générale (V) 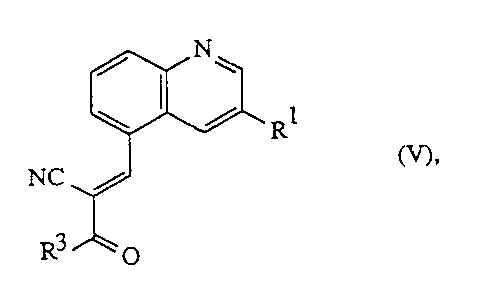 dans laquelle
R¹ est un groupe aryle ayant 6 à 10 atomes de carbone, qui est substitué, le cas échéant jusqu'à 3 fois identiques ou différentes, par un radical halogéno, nitro, cyano, trifluorométhyle, trifluorométhoxy, trifluorométhylthio,
ou par un radical alkyle linéaire ou ramifié ayant jusqu'à 8 atomes de carbone, qui peut être substitué de son côté par un radical aryle ayant 6 à 10 atomes de carbone,
ou substitué par un radical alkoxy ou alkoxycarbonyle linéaire ou ramifié ayant chacun jusqu'à 8 atomes de carbone, carboxy, amino, ou par un groupe de formule -NR⁴R⁵,
dans laquelle
R⁴ et R⁵ sont identiques ou différents et représentent de l'hydrogène, un radical alkyle linéaire ou ramifié ayant jusqu'à 8 atomes de carbone, phényle ou benzyle,
ou un radical thiényle, et
R³ représente de l'hydrogène ou un groupe alkyle linéaire ou ramifié ayant jusqu'à 8 atomes de carbone,
caractérisé en ce qu'on fait réagir des composés de formule générale (VII) 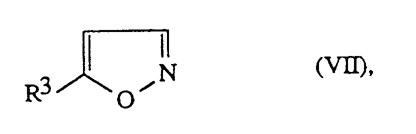 dans laquelle
R³ a la définition indiquée dans la revendication 6,
avec des hydroxydes ou alcoolates alcalins pour former les sels alcalins des composés de formule générale (VIII)
R³-CO-CH₂-CN (VIII)
dans laquelle
R³ a la définition indiquée dans la revendication 6,
le cas échéant en présence de solvants organiques inertes et en la présence éventuelle d'acides, de préférence d'acide acétique, et on fait réagir ces sels avec des aldéhydes de formule générale (II) 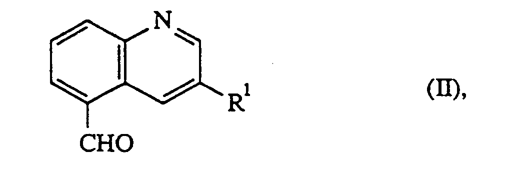 dans laquelle
R¹ a la définition indiquée dans la revendication 6,
à des températures comprises entre 0°C et 150°C dans des solvants organiques inertes avec addition d'acide et, le cas échéant en présence d'un catalyseur tel que l'acétate de pipéridine.

3. Procédé de production de médicaments, caractérisé en ce qu'on fait prendre une forme d'administration appropriée à au moins un composé de formule générale (I) suivant la revendication 1, en utilisant, le cas échéant, des substances auxiliaires et des supports classiques.
